(19) Europäisches Patentamt
European Patent Office
Office européen des brevets

(11) **EP 2 365 974 B1**

(12) **EUROPEAN PATENT SPECIFICATION**

(45) Date of publication and mention of the grant of the patent:
**25.12.2013 Bulletin 2013/52**

(21) Application number: **08877998.8**

(22) Date of filing: **06.11.2008**

(51) Int Cl.:
***A61K 31/428*** $^{(2006.01)}$ ***C07D 277/66*** $^{(2006.01)}$
***A61K 51/04*** $^{(2006.01)}$ ***A61K 101/02*** $^{(2006.01)}$
***C07B 59/00*** $^{(2006.01)}$

(86) International application number:
**PCT/KR2008/006546**

(87) International publication number:
**WO 2010/053218 (14.05.2010 Gazette 2010/19)**

(54) **FLUORINATED BENZOTHIAZOLE DERIVATIVES, PREPARATION METHOD THEREOF AND IMAGING AGENT FOR DIAGNOSING ALTZHEIMER'S DISEASE USING THE SAME**

FLUORIERTE BENZOTHIAZOLDERIVATE, VERFAHREN ZU DEREN HERSTELLUNG UND BILDDARSTELLENDES MITTEL FÜR DIE DIAGNOSE VON ALZHEIMER-KRANKHEIT, WELCHES DIESE VERWENDET

DÉRIVÉS DE BENZOTHIAZOLE FLUORÉS, PRÉPARATION DE CEUX-CI ET AGENT D'IMAGERIE UTILISANT CES DÉRIVÉS POUR DIAGNOSTIQUER LA MALADIE D'ALZHEIMER

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**21.09.2011 Bulletin 2011/38**

(73) Proprietor: **SNU R&DB Foundation**
**Seoul 151-742 (KR)**

(72) Inventors:
- **KIM, Sang Eun**
  **Seoul 135-240 (KR)**
- **LEE, Byung Chul**
  **Seoul 143-819 (KR)**
- **KIM, Ji Sun**
  **Anyang-si**
  **Gyeonggi-do 430-705 (KR)**
- **CHUN, Young Sin**
  **Seoul 134-790 (KR)**

(74) Representative: **Brady, Paul Andrew**
**Abel & Imray**
**20 Red Lion Street**
**London WC1R 4PQ (GB)**

(56) References cited:
- **GJERMUND HENRIKSEN ET AL: "Metabolically Stabilized Benzothiazoles for Imaging of Amyloid Plaques", JOURNAL OF MEDICINAL CHEMISTRY, vol. 50, no. 6, 1 March 2007 (2007-03-01), pages 1087-1089, XP55028870, ISSN: 0022-2623, DOI: 10.1021/jm061466g**
- **BROWN G D ET AL: "RADIOLABELLING OF THE POTENTIAL ANTI-CANCER AGENT, 5-FLUORO-2-(4-AMINO-3-METHYLPHENYL)BENZOTH IAZOLE (5F203) WITH FLUORINE-18", JOURNAL OF LABELLED COMPOUNDS AND RADIOPHARMACEUTICALS, JOHN WILEY, CHICHESTER, GB, vol. 44, no. SUPPL. 01, 1 January 2001 (2001-01-01), XP009010853, ISSN: 0362-4803**
- **PIKE V W ET AL: "Facile synthesis of substituted diaryliodonium tosylates by treatment of aryltributylstannane with Koser's reagent", JOURNAL OF THE CHEMICAL SOCIETY, PERKIN TRANSACTIONS 1, CHEMICAL SOCIETY, LETCHWORTH; GB, vol. 1, no. 3, 1 January 1999 (1999-01-01) , pages 245-248, XP003016179, ISSN: 0300-922X, DOI: 10.1039/A809349K**
- **ZHENG, M-Q ET AL.: 'Syntheses and evaluation of fluorinated benzothiazole anilines as potential tracers for beta-amyloid plagues in Alzheimer's disease.' JOURNAL OF FLUORINE CHEMISTRY. vol. 129, no. 3, 2008, pages 210 - 216, XP022473506**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KIM MK ET AL.: '3D-QSAR of PET agents for imaging beta-amyloid in Alzheimer's disease.' BULLETIN OF THE KOREAN CHEMICAL SOCIETY. vol. 28, no. 7, 2007, pages 1231 - 1234, XP008135837
- HUTCHINSON I ET AL.: 'Antitumor benzothiazoles. 14. Synthesis and in vitro biological properties of fluorinated 2-(4-aminophenyl)benzothiazoles.' JOURNAL OF MEDICINAL CHEMISTRY vol. 44, no. 9, 2001, pages 1446 - 1455, XP002215317
- MATHIS CA. ET AL.: 'Synthesis and evaluation of 11C-labeled 6-substituted 2-arylbenzothiazoles as amyloid imaging agents.' JOURNAL OF MEDICINAL CHEMISTRY vol. 46, no. 13, 2003, pages 2740 - 2754, XP002285914
- KLUNK WE. ET AL.: 'Uncharged thioflavin-T derivatives bind to amyloid-beta protein with high affinity and readily enter the brain.' LIFE SCIENCES. vol. 69, 2001, pages 1471 - 1484, XP002189042

## Description

BACKGROUND OF THE INVENTION

1. Field of the Invention

The present disclosure relates to fluorinated benzothiazole derivatives, a preparation method thereof, and an imaging agent for diagnosing Alzheimer's disease using the same.

2. Description of the Related Art

At the turn of this century, with the population gradually aging due to increased human life expectancy, degenerative brain diseases such as Alzheimer's disease are becoming an important public health issue. The prevalence rate of Alzheimer's disease is 1% in people in their sixties and 20% to 30% in the elderly up to 85 years old. Along with the rise in the prevalence rate due to the increase in life expectancy, the serious progression of the disease and the need for prolonged treatment cause not only psychological and economic hardship to patients and their families, but also much damage to society.

So far, Alzheimer's disease has been diagnosed from clinical symptoms such as reduction in cognitive capabilities, irreversible amnesia, loss of directional sense, dyslogia, etc., or from reduction in glucose metabolism in parietal lobe areas using [$^{18}$F]fluorodeoxyglucose (FDG). However, the recent direct visualization of beta-amyloid plaques of a degenerative brain allows for a more accurate diagnosis.

Beta-amyloid plaques were defined by ADNI (Alzheimer's Disease Neuroimaging Initiative) organized by the US NIA (National Institute of Aging) in 2004 as the most potent biomarker for Alzheimer's disease. Accordingly, the quantification of beta-amyloid plaque accumulation using noninvasive *in vivo* molecular imaging may be a technology with which an epochal development in early diagnosis and treatment of Alzheimer's disease may be brought about. Methods for allowing for the visualization of beta-amyloid plaques from living individual cells include single photon emission computed tomography (SPECT) or positron emission tomography (PET) as a nuclear medicinal analysis method.

Radiopharmaceuticals to be used will be used at a very small concentration in which pharmacological effects are ruled out, and to visualize beta-amyloid plaques, a significant amount of the pharmaceutical should be introduced into the brain. Then, it should cross the blood brain barrier (hereinafter, 'BBB'). Thus, in order for radiopharmaceuticals to be used for diagnosis of Alzheimer's disease to cross the BBB, a lipophilicity sufficient for diffusion into the cell membrane should be present. In addition, the uptake of ideal radiopharmaceuticals for diagnosis of Alzheimer's disease should rapidly occur in a normal human brain, and then they should be released *ex vivo* in a short time without any interference with the metabolism.

Among compounds known so far, a Pittsburgh Compound-B (hereinafter, 'PIB') labeled with carbon-11, a radioactive isotope, is a marker to identify beta-amyloid plaque deposition in the brain for diagnosis of Alzheimer's disease and is known as the most potent compound among derivatives of benzothiazoles (hereinafter, 'BTA') (Mathis, C.A., Wang. Y., Holt, D. P., Huang, G-F., Debnath, M.L., Klunk, W.E., J. Med. Chem. 2003, 46:2740-2754; Cai, L., Innis, R. B., Pike, V. W., Curr. Med. Chem. 2007, 14(1):19-52). The structures of a variety of conventionally known radioactive isotope-labeled BTA derivatives are shown in the following Table 1.

<Table 1>

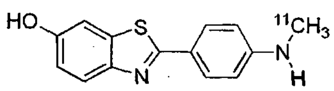

**[N-methyl-$^{11}$C]6-Me-BTA-1**

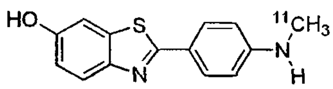

**[$^{11}$C]6-OH-BTA-1, PIB**

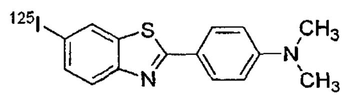

**[$^{125}$I]TZDM**

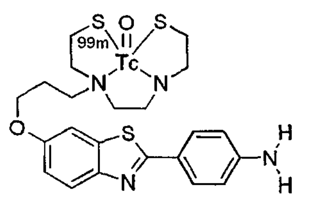

**[$^{99m}$Tc]6-BTA**

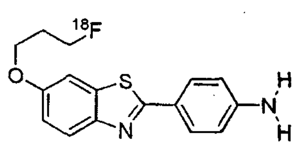

**[$^{18}$F]6-$C_3H_6$F-BTA**

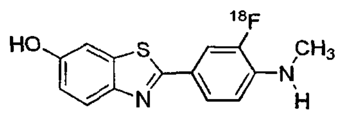

**[$^{18}$F]AH110690**

The PIB not only binds strongly to a beta-amyloid, but also is known to have the highest brain uptake/elimination

rate among beta-amyloid imaging agents currently developed. However, C-11 with a short half-life should be used for synthesis of the PIB, and because the marking method is so complicated and its productivity is so low, it is impossible to synthesize the compound in the absence of a cyclotron which can produce C-11.

To solve these problems, much research has been conducted on BTA derivatives labeled with iodine-125 and technetium-99m as different kinds of radioactive isotopes as described in Table 1. However, because [$^{125}$I]TZDM remains too long in a normal brain and a [99mTc]6-BTA labeled with technetium-99m is too big to cross the BBB of the brain and too polar, it is more difficult to obtain a better brain image than with the PIB.

Much research has been conducted on the development of BTA radiopharmaceuticals labeled with fluorine-18 using various derivatives. For example, because it is too difficult to label a fluorine-18 directly to the ring of an aromatic compound, a fluorine-18 labeled BTA derivative was developed through the O-alkylation at a hydroxyl group in position 6 of the BTA ring, showing poor results (refer to [$^{18}$F]6-$C_3H_6$F-BTA in Table 1).

Recently, a fluorine-18 labeled compound ([$^{18}$F]AH110690 in Table 1, GE Healthcare) in position 3 of the right phenyl of the PIB compound has been developed and is known to be under clinical experiments in Europe since 2008. However, because the compound has a low yield in fluorine-18 labeling of the aromatic ring and the labeling process includes three or more steps, the compound with a 110-minute half life has problems in terms of productivity. However, a method of direct fluorine-18 labeling of the aromatic ring may be a result showing that it corresponds to the development strategy of fluorine-18 labeled BTA compounds which will substitute for [$^{11}$C]PIB, and so far there has been no case reported of a direct fluorine-18 labeling of the BTA itself, and not of the right phenyl.

Thus, the present inventors have synthesized fluorinated BTA derivatives with an excellent bonding force to beta-amyloid plaques as a potent biomarker for Alzheimer's disease and precursors of BTA derivatives which enable direct fluorine-18 labeling of the BTA, confirmed in ex vivo experiments that these BTA derivatives have excellent binding affinity and lipophilicity to beta-amyloid plaques, recognized through in vivo cerebral uptake and elimination rates in normal mice and brain imaging photos in normal humans that they are materials with which diagnostic imaging of Alzheimer's disease is possible, and have made the present invention.

## SUMMARY OF THE INVENTION

One object of the present invention is to provide fluorinated benzothiazole derivatives represented by Chemical Formula 1.

Another object of the present invention is to provide a method for preparing the fluorinated benzothiazole derivatives.

Still another object of the present invention is to provide precursors of benzothiazole derivatives represented by Chemical Formula 2.

Even another object of the present invention is to provide a method for preparing the precursors of benzothiazole derivatives.

Yet another object of the present invention is to provide an imaging agent for diagnosing Alzheimer's disease using fluorinated benzothiazole derivatives represented by Chemical Formula 1.

Further another object of the present invention is to provide an imaging agent for diagnosing Alzheimer's disease.

In order to achieve the objects, the present invention provides fluorinated benzothiazole derivatives represented by Chemical Formula 1, precursors of these derivatives represented by Chemical Formula 2, and methods for synthesizing them, represented by Reaction Formulas 1 to 3.

The present invention also provides an imaging agent for diagnosing Alzheimer's disease using derivatives of Chemical Formula 1, which bind strongly to beta-amyloid plaques as a biomarker for Alzheimer's disease and are highly efficient in terms of cerebral uptake and elimination, and a diagnosis method thereof.

According to the present invention, fluorine-labeled benzothiazole derivatives, which have been difficult to synthesize by conventional methods, may be obtained by simple processes and the thus-obtained benzothiazole derivatives may be useful in diagnosing the presence and severity of Alzheimer's disease.

## BRIEF DESCRIPTION OF THE DRAWINGS

The above and other objects, features and other advantages of the present invention will be more clearly understood from the following detailed description taken in conjunction with the accompanying drawings, in which:

FIG. 1 is a graph illustrating results of a normal mouse's in vivo cerebral uptake and release according to Experimental Example 1.3 on compounds of Examples 1, 2, and 3 as derivatives of Chemical Formula 1 of the present invention.

FIGs. 2, 3, and 4 are a group of photographs including brain images captured over 2 hours according to Experimental Example 1.4 on compounds of Examples 1, 2, and 3 as derivatives of Chemical Formula 1 of the present invention.

DESCRIPTION OF THE PREFERRED EMBODIMENTS

Features and advantages of the present invention will be more clearly understood by the following detailed description of the present preferred embodiments by reference to the accompanying drawings. It is first noted that terms or words used herein should be construed as meanings or concepts corresponding with the technical sprit of the present invention, based on the principle that the inventor can appropriately define the concepts of the terms to best describe his own invention. Also, it should be understood that detailed descriptions of well-known functions and structures related to the present invention will be omitted so as not to unnecessarily obscure the important point of the present invention.
Hereinafter, the present invention will be described in detail.
The present invention provides fluorinated benzothiazole derivatives represented by Chemical Formula 1.

【Chemical Formula 1】

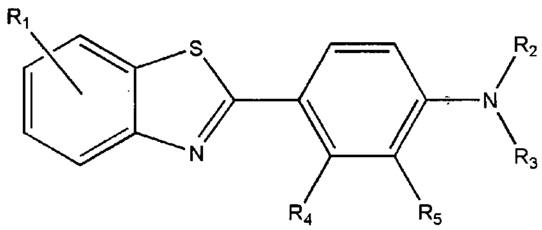

Where,
$R_1$ is $^{18}F$, and $R_1$ is substituted into one in 4, 5, 6, and 7 positions of the benzothiazole ring;
$R_2$ is one selected from the group consisting of hydrogen, $C_1$-$C_4$ linear or branched alkyl, $C_1$-$C_4$ linear or branched alkylcarbonyl, 2-(2'-methoxy-(ethoxy)$_n$)$C_1$-$C_4$ linear or branched alkylcarbonyl, and 2-(2'-methoxy-(ethoxy)$_n$)$C_1$-$C_4$ linear or branched alkyl, wherein n is an integer of 1 to 5;
$R_3$ is hydrogen, or $C_1$-$C_4$ linear or branched alkyl; and
$R_4$ and $R_5$ are each independently hydrogen or hydroxy. Preferably, $R_2$ is hydrogen, methyl, acetyl, 2-(2'-methoxy-(ethoxy)$_n$)acetyl or 2-(2'-methoxy-(ethoxy)$_n$)ethyl, and n is an integer of 1 to 5; and
$R_3$ is hydrogen or methyl.
More preferably, the derivative of Chemical Formula 1 according to the present invention is one selected from the group consisting of

1) 6-[$^{18}$F]fluoro-2-(4'-aminophenyl)benzothiazole;
2) 6-[$^{18}$F]fluoro-2-(4'-N-methylaminophenyl)benzothiazole;
3) 6-[$^{18}$F]fluoro-2-(4'-N,N-dimethylaminophenyl)benzothiazole.

Also disclosed herein are

4) 6-fluoro-2-(4'-aminophenyl)benzothiazole;
5) 6-fluoro-2-(4'-N-methylaminophenyl)benzothiazole;
6) 6-fluoro-2-(4'-N,N-dimethylaminophenyl)benzothiazole;
7) 6-fluoro-2-(4'-N-acetamidephenyl)benzothiazole;
8) 6-fluoro-2-(4'-N-(2"-(2"-methoxyethoxy)acetamidephenyl))benzothiazole;
9) 6-fluoro-2-(4'-N-(2"-(2"-(2"-methoxyethoxy)ethoxy)acetamidephenyl))benzothiazol e; and
10) 6-fluoro-2-(4'-N-(2"-(2"-methoxyethoxy)ethoxyaminophenyl))benzothiazole.

Hereinafter, a method for preparing fluorinated benzothiazole derivatives of Chemical Formula 1 will be described.
As indicated in the following Reaction Formula 1, the present invention provides a method (preparation method 1) for preparing fluorinated benzothiazole derivatives of Chemical Formula 1, the method including
a mixture of [$^{18}$F]fluorine and tetrabutylammoniumcarbonate (TBA) is used and reacted with a compound of Chemical Formula 2 to label the $^{18}$F directly to the benzothiazole ring.

<Reaction Formula 1>

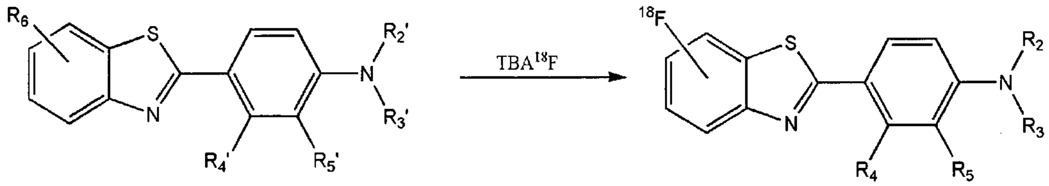


2 1a

Where,

$R_2$, $R_3$, $R_4$, and $R_5$ are the same as defined for the compound of Chemical Formula 1 above;

$R_6$ is iodophenyltosylate (

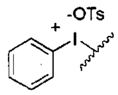

), 2-iodothiophenetosylate (

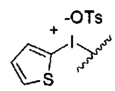

), or 2-iodoresinthiophenetosylate (

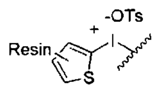

);

$R_{2'}$ is one selected from the group consisting of the $R_2$ substituents as defined for the compound of Chemical Formula 1, and oxygen, $R_{3'}$ is one selected from the group consisting of the $R_3$ substituents as defined for the compound of Chemical Formula 1, t-butoxycarbonyl (Boc) and oxygen, and

when one of $R_{2'}$ and $R_{3'}$ is hydrogen, the other is also hydrogen, and when $R_{3'}$ is t-butoxycarbonyl (BOC), $R_3$ is hydrogen; and

$R_4$, and $R_5$, are each independently one selected from the group consisting of hydrogen and methoxymethyl (MOM) ether; and, if necessary, carrying out a reduction alkylation, deprotection and/or acylation reaction to produce said compound of Chemical Formula 1.

In the preparation method 1 according to the present invention, the fluorinating of $^{18}$F may be performed through a process, the process including a mixture of [$^{18}$F]fluorine and TBA is introduced into a vacutainer and nitrogen gas is blown at 75°C to 85°C into the container to dry the [$^{18}$F]fluorine (Step 1); and the dried [$^{18}$F]fluorine in Step 1 is transferred to a reaction vessel in which a starting material of Chemical Formula 2 as described in Reaction Formula 1 and 2,2,6,6-tetramethylpiperidine-N-oxyl (TEMPO) are dissolved in a acetonitrile/water solvent, followed by irradiation of microwave onto the reaction vessel (Step 2).

Additionally, fluorine-18-fluorinated benzothiazole derivatives may be separated/purified by performing step 2 and then cooling at room temperature, followed by high-performance liquid chromatography (HPLC). If necessary, an appropriate reaction, for example, reduction, alkylation, deprotection, acylation, etc. may be also carried out to introduce a substituent included in the range of derivatives of Chemical Formula 1 according to the present invention.

In the preparation method 1 according to the present invention, because the compound in Chemical Formula 2 used as a starting material is a material in which iodophenyltosylate (

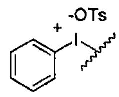

), 2-iodothiophenetosylate (

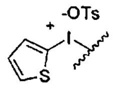

), 2-iodoresinthiophenetosylate (

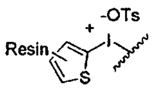

), etc. is substituted into the benzene ring of the benzothiazole, the compound has a low relative electron density of the benzothiazole ring between the two aromatic groups at the iodine center. As a result, it may allow the fluorine-18 to be directly substituted for the benzothiazole ring and increase the yield and selectivity.

As indicated in the following Chemical Formula 2, the present application discloses another method (preparation method 2) for preparing fluorinated benzothiazole derivatives represented by Chemical Formula 1b, the method including a coupling reaction is carried out between a compound (3) and a compound (4) in pyridine solvent to prepare a compound (5) (Step 1); the compound (5) is reacted with a Lawesson's reagent in toluene solvent to prepare a compound (6) (Step 2); the compound (6) was reacted with potassium ferricyanide ($K_3Fe(CN)_6$) to prepare a compound (7) in which a benzothiazole ring is introduced (Step 3); and the nitro group of the compound (7) is modified to prepare a compound (1b) in which $R_2$ and $R_3$ are substituted (Step 4).

<Reaction Formula 2>

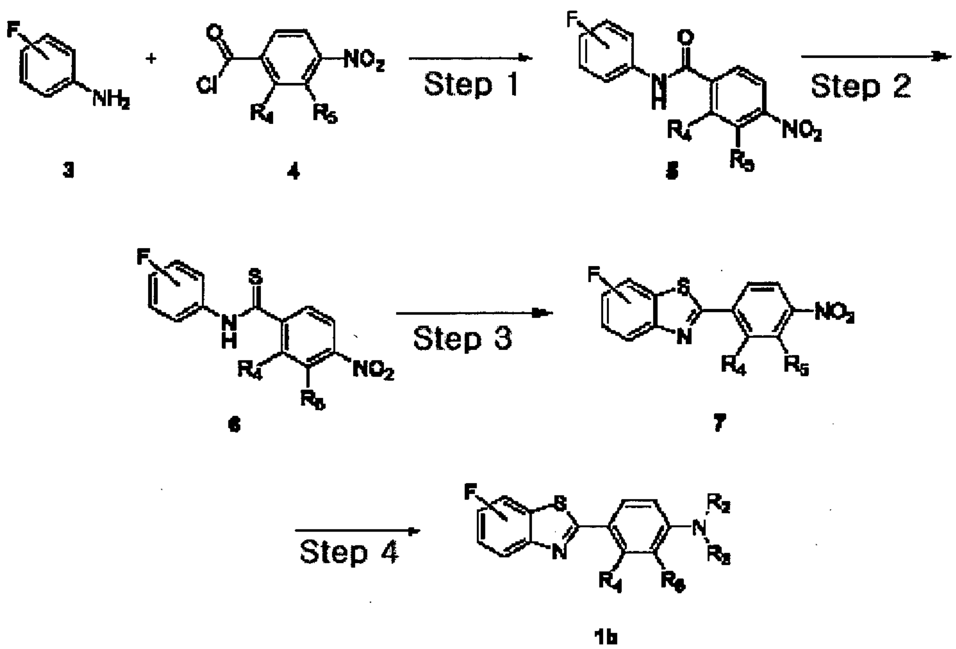


(where, a compound of Chemical Formula 1b is a kind of benzothiazole derivative of Chemical Formula 1, and $R_2$, $R_3$, $R_4$, and $R_5$ are the same as defined in Chemical Formula 1 and F is $^{18}F$ or $^{19}F$.)

In the preparation method 2 according to the present invention, in order to introduce or modify $R_2$ and $R_3$, the substituents of the benzothiazole of Chemical Formula 1, reduction of a nitro group, alkylation or acylation of a amine

group produced by the reduction, reduction of a carbonyl group produced by the acylation, etc. may be appropriately performed.

In the preparation method 2 disclosed herein, intermediates obtained in each step may be separated/purified by a filtering method, a purification method, etc. known in the art of organic synthesis.

Furthermore, the present invention provides benzothiazole precursors represented by the following Chemical Formula 2.

【Chemical Formula 2】

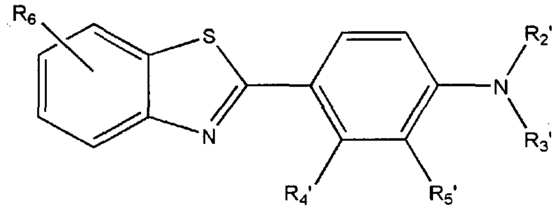

where, $R_6$, $R_{2'}$, $R_{3'}$, $R_{4'}$, and $R_5$, are the same as defined in Reaction Formula 1.

The benzothiazole precursors of Chemical Formula 2 may be used as a starting material which prepares derivatives of Chemical Formula 1. $R_6$ induces a relatively low electron density to the benzothiazole ring compared to the opposite aromatic compound at the iodine center both to allow the fluorine-18 to be directly introduced into the benzothiazole ring, and to increase the yield and selectivity.

Preferably, the benzothiazole derivatives of Chemical Formula 2 according to the present invention may be selected from the group consisting of:

(a) 6-iodophenyl-2-(4'-nitrophenyl)benzothiazoleiodoniumtosylate;
(b) 6-iodophenyl-2-(4'-N-methyl(t-butyloxycarbonyl)aminophenyl)benzothiazoleiodoniumtosylat e; and
(c) 6-iodophenyl-2-(4'-N,N-dimethylaminophenyl)benzothiazoleiodoniumtosylate.

As indicated in the following Reaction Formula 3, the present invention also provides a method (preparation 3) for preparing benzothiazole precursors of Chemical Formula 2, the method including a $-R_6$ group is introduced into the benzothiazole ring of a compound (8).

<Reaction Formula 3>

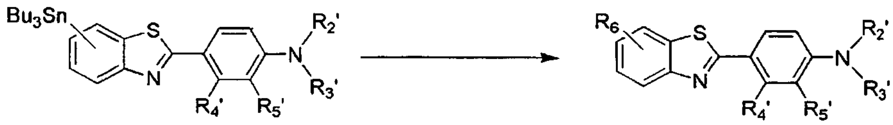

8 2

where, $R_6$, $R_{2'}$, $R_{3'}$, $R_{4'}$, and $R_5$, are the same as defined in Reaction Formula 1.

The preparation method 3 according to the present invention may be performed by using hydroxytosyloxyiodobenzene (Koser's reagent) with a high electron density, 2-hydroxytosyloxyiodothiophene, 2-hydroxytosyloxyiodothiophene bound to resin, etc. as a reactant with a compound (8) for introduction of a $-R_6$ group.

This reaction may be performed by dissolving a compound for introduction of the $-R_6$ group in acetonitrile solvent under inert gas atmosphere, dripping the compound (8) dissolved in methylene chloride at 0°C or less, and stirring the compound at room temperature for 12 to 15 hours.

2-hydroxytosyloxyiodothiophene bound to the resin may be linked to the thiophene in the form of a covalent bond using an alkyl or PEG linker, and the resin may include a polymer such as polystyrene, polyacrylamide, polypropylene, etc. When a compound for introduction of the $-R_6$ group bound to the resin is used, a labeling compound may be obtained without further separation process after a labeling in the fluorine labeling process, resulting in a simplification in the labeling process of a fluorine-18 with a 110 minute-half life and a high radiochemical yield.

Furthermore, the present invention provides an imaging agent for use in diagnosing Alzheimer's disease using

benzothiazole derivatives of Chemical Formula 1.

The fluorinated benzothiazole derivatives of Chemical Formula 1 according to the present invention may be used as a positron emission tomography (PET) radioactive tracer by forming a bond with *in vivo* beta-amyloid plaques. The positron emitted after bonding with the beta-amyloid plaques may annihilate with a contiguous electron present *in vivo,* and two gamma energies (511 keV) then produced may be collected to enable a direct visualization of beta-amyloid plaques through PET.

In the benzothiazole derivatives of Chemical Formula 1, $R_2$ and $R_3$ substituted in the amine group, and $R_4$ and $R_5$ substituted in position 2 of the benzothiazole may be variously modified to control the cerebral uptake and release of the benzothiazole derivatives and the lipophilicity of beta-amyloid plaques. If necessary, the polarity of these substituents may be increased to increase the release rate in a normal brain.

Thus, the benzothiazole derivatives of Chemical Formula 1 according to the present invention may be administered to mammals, preferably humans to be useful in diagnosis of the presence and severity of Alzheimer's disease.

Hereinafter, the present invention will be described in more detail with reference to the following examples 1 to 3 and 11 to 13. However, the following examples are provided for illustrative purposes only, and the scope of the present invention should not be limited thereto in any manner.

Preparation Example: Preparation of the benzothiazole derivatives of Chemical Formula 2 according to the present invention as a starting material

<Preparation Example 1> Preparation of 6-tributylstannyl-2-(4'-nitrophenyl)benzothiazole

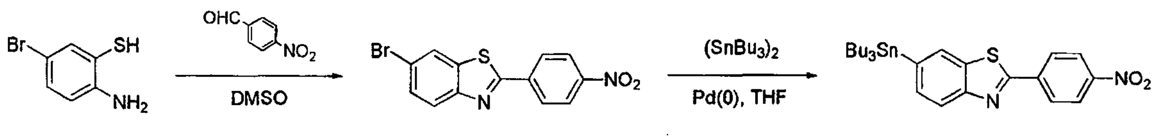


9

(Step 1) Preparation of 6-bromo-2-(4'-nitrophenyl)benzothiazole

A mixture of 2-amino-5-bromobenzenethiol (312 mg, 1.53 mmol) and 4-nitrobenzaldehyde (231 mg, 1.53 mmol) was dissolved in dimethylsulfoxide (DMSO)(3 mℓ) and then the solution was stirred at 180 °C for 30 minutes. After the reaction container was cooled at room temperature, iced water was introduced into the container and the resulting mixture was distilled under reduced pressure to obtain a precipitate. A solution of tetrahydrofuran (1.5 mℓ) and methanol (MeOH)(30 mℓ) was used for recrystallization of the thus-obtained compound, and the mixture was filtered under reduced pressure to obtain a target compound.

A yellow solid; mp = 192.4-193.0 °C; $^1H$ NMR (300 MHz, DMSO-$d_6$) δ 8.55 (d, *J* = 2.1 Hz, 1H), 8.38 (dd, *J* = 9.3 Hz, *J* = 9.0 Hz, 2H), 8.08 (d, *J* = 8.7 Hz, 1H), 7.76 (dd, *J* = 8.7 Hz, *J* = 2.1, 1H); $^{13}C$ NMR (100 MHz, DMSO-$d_6$) δ 165.87, 152.49, 148.92, 137.92, 137.02, 130.34, 128.51, 125.27, 124.98, 124.64, 123.58, 119.10; MS (EI) *m/z* 336 ($M^+$, $^{81}Br$), 334 ($M^+$, $^{79}Br$); Anal. ($C_{13}H_7BrN_2O_2S$) calcd: C, 46.58; H, 2.11; N, 8.36; S, 9.57. found: C, 46.72; H, 2. 06; N, 8.38; S, 9.56.

(Step 2) Preparation of 6-tributylstannyl-2-(4'-nitrophenyl)benzothiazole

A mixture of the compound (190 mg, 0.57 mmol) obtained in Step 1, bistributyltin (630 μℓ, 1.26 mmol), and tetrakis(triphenylphosphine)-palladium(0) was dissolved in anhydrous tetrahydrofuran (10 mℓ) under argon gas, followed by stirring at 90°C for 12 hours.

To a mixture of 2-(4'-nitrophenyl)-6-bromobenzothiazole (190 mg, 0.57 mmol) and tetrakis(triphenylphosphine)-palladium(0) (Mg, 0.06 mmol) in dry tetrahydrofuran (10 mL) was added and bistributyltin (630 μℓ, 1.26 mmol) in dry tetrahydrofuran (10 mL) under argon gas at room temperature.

The reaction mixture was heated at 90 °C for 12 h. At the end of the reaction, the reaction mixture was cooled to room temperature and filtered by celite. The crude product was purified by flash chromatography (silica gel, 80:20 hexane-ethyl acetate) to give 161 mg (52%) of 2-(4'-nitrophenyl)-6-tributylstannylbenzothiazole.

As a pale yellow oil; $^1H$ NMR (300 MHz, $CDCl_3$) δ 8.35 (d, *J* = 9.0 Hz, 2H), 8.27 (d, *J* = 9.0 Hz, 2H), 8.09-8.03 (m, 2H), 7.62 (dd, *J* = 8.1, 0.4 Hz, 1H), 1.57-1.52 (m, 6H), 1.41-1.29 (m, 6H) 1.16-1.10 (m, 6H), 0.92-0.85 (m, 9H); $^{13}C$ NMR (100 MHz, $CDCl_3$) δ 164.39, 154.18, 149.17, 141.20, 139.63, 135.84, 134.52, 128.48, 128.42, 124.50, 123.37, 29.28, 27.57, 13.87, 10.13; MS (FAB) *m/z* 547 ($M^+$ + H); Anal. ($C_{25}H_{34}N_2O_2SSn$) calcd: C, 55.06; H, 6.28; N, 5.14; S,

5.88. found: C, 55.05; H, 6.28; N, 5.11; S, 5.88.

<Preparation Example 2> Preparation of 6-tributylstannyl-2-(4'-N,N-dimethylaminophenyl)benzothiazole

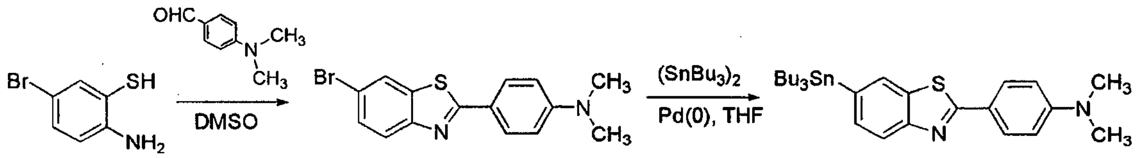


**10**

A target compound was obtained in the same way as in Preparation Example 1, except that 4-(N,N-dimethylamino)benzaldehyde was used as a starting material instead of 4-nitrobenzaldehyde.

2-(4'-N,N-Dimethylaminophenyl)-6-bromobenzothiazole; A yellow solid; mp = 208.6-208.7 °C; $^1H$ NMR (300 MHz, $CDCl_3$) δ 7.96-7.91 (m, 3H), 7.81 (d, *J* = 8.7 Hz, 1H), 7.52 (dd, *J* = 8.7, 2.0 Hz, 1H), 6.74 (d, *J* = 9.0 Hz, 2H), 3.06 (s, 6H); $^{13}C$ NMR (100 MHz, $CDCl_3$) δ 169.46, 154.36, 153.49, 136.37, 129.52, 129.06, 123.99, 123.42, 120.96, 117.53, 111.80, 40.30; MS (EI) *m/z* 334 ($M^+$, $^{81}Br$), 332 ($M^+$, $^{79}Br$); Anal. ($C_{15}H_{13}BrN_2S$) calcd: C, 54.06; H, 3.93; N, 8.41; S, 9.62. found: C, 54.05; H, 3.91; N, 8.45; S, 9.62.

2-(4'-Nitrophenyl)-6-tributylstannylbenzothiazole; A yellow oil; $^1H$ NMR (400 MHz, $CDCl_3$) δ 7.98-7.92 (m, 4H), 7.50 (d, *J* = 7.6 Hz, 1H), 6.75 (d, *J* = 8.4 Hz, 2H), 3.06 (s, 6H), 1.58-1.52 (m, 6H), 1.39-1.32 (m, 6H), 1.12-1.08 (m, 6H), 0.91-0.88 (m, 9H); $^{13}C$ NMR (100 MHz, $CDCl_3$) δ 168.44, 154.53, 152.36, 137.78, 135.00, 133.71, 129.08, 129.03, 121.97, 121.82, 111.91, 40.40, 29.21, 27.60, 13.89, 10.04; MS (EI) *m/z* 544 ($M^+$); Anal. ($C_{27}H_{40}N_2SSn$) calcd: C, 59.68; H, 7.42; N, 5.16; S, 5.90. found: C, 59.65; H, 7.37; N, 5.22; S, 5.94.

<Preparation Example 3> Preparation of 2-(4'-*N*-tert-Butyloxycarbonyl-methylaminophenyl)-6-tributylstannylbenzothiazole

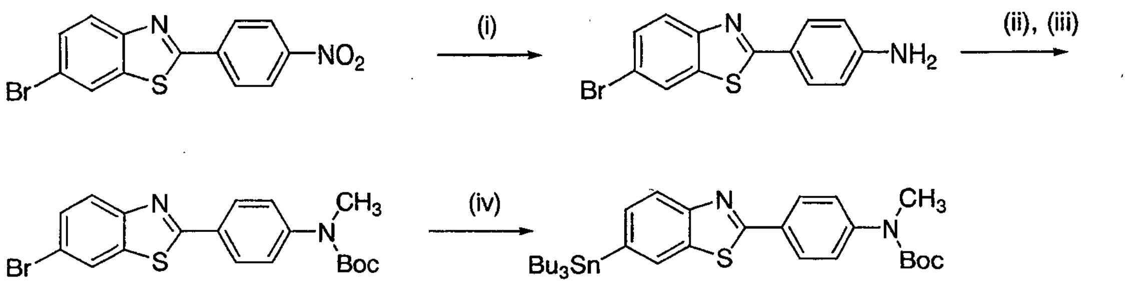


Reagents : (i) $SnCl_2$, EtOH, 90 °C, 2 h; (ii) a) formaldehyde, MeOH, reflux, 2 h; b) $NaCNBH_3$, AcOH, room temp., 1.5 h; (iii) $Boc_2O$, THF, 85 °C, 12 h; (iv) $Sn_2Bu_3$, Pd(0), tetrahydrofuran, 85 °C, 8 h;

(Step 1): Preparation of 2-(4'-aminophenyl)-6-bromobenzothiazole

To a solution of 2-(4'-nitrophenyl)-6-bromobenzenethiol (1.1 g, 3.29 mmol), was obtained in preparation Example 1, in ethanol (50 mL) was added tin(II) chloride e (4.92 mg, 26.3 mmol). The reaction mixture was heated at 100 °C under nitrogen gas for 1 h. Ethanol was removed by evaporator, and the residue was dissolved in ethyl acetate (100 mL), the organic solution was washed with saturated sodium bicarbonate (30 mL) followed by water (30 mL) and dried over sodium sulfate. The crude product was purified by flash choromatography (silica gel, 70:30 hexane-ethyl acetate) to give 903 mg (90%) of 2-(4'-aminophenyl)-6-bromobenzothiazole

A yellow solid; mp = 219.3-221.0 °C; $^1H$ NMR (400 MHz, DMSO-$d_6$) δ 8.23 (d, *J* = 1.8 Hz, 1H), 7.87 (d, *J* = 8.7 Hz, 2H), 7.82 (d, *J* = 8.7 Hz, 1H), 7.53 (dd, *J* = 8.4, 1.8 Hz, 1H), 6.73 (d, *J* = 8.7, 2.0 Hz, 2H), 4.03 (brs, 2H, $NH_2$); MS

(CI) m/z 306 ($M^+$ + H); $^{13}C$ NMR (75 MHz, $CDCl_3$) δ 149.50, 149.48, 136.24, 129.48, 129.19, 123.92, 123.52, 123.43, 117.76, 117.73, 114.76; MS (FAB) *m/z* 307 ($M^+$ + H, $^{81}Br$), 305 ($M^+$ + H, $^{79}Br$) Anal. ($C_{13}H_9BrN_2S$) calcd: C, 51.16; H, 2.97; N, 9.18; S, 10.51. found: C, 51.04; H, 3.03; N, 9.03; S, 10.69.

(Step 2): Preparation of 2-(4'-*N*-*tert*-Butyloxycarbonyl-methylaminophenyl)-6-bromobenzothiazole

To a solution of 2-(4'-*N*-aminophenyl)-6-bromobenzenethiol (300 mg, 0.98 mmol) in methanol (20 mL) was added formaldehyde (220 μL, 2.96 mmol). The reaction mixture was refluxed for 1.5 h. Ethanol was removed by evaporator and vacuum. The residue was dissolved in methanol (100 mL) again, and was added sodium cyanoborohyde (247 mg, 3.92 mmol) and acetic acid (6 μL, pH 6) slowly. The reaction mixture was stirred at room temperature for 1.5 h. Methanol was removed by evaporator and the residue was dissolved in ethyl acetate (50 mL), the organic solution was washed with saturated sodium bicarbonate (30 mL) followed by saline (30 mL) and dried over sodium sulfate. The crude product was purified by flash chromatography (silica gel, 70:30 hexane-ethyl acetate). To the obtained 2-(4'-methylaminophenyl)-6-bromobenzothiazole in tetrahydrofuran (15 mL) was added di-*tert*-butyl-dicarbonate (217 mg, 0.96 mmol) at 0 °C. The reaction mixture was refluxed for 12 h. At the end of the reaction, the reaction mixture was cooled to room temperature and poured into ice-water (20 mL) and ethyl acetate (40 mL). The organic solution was washed with saturated sodium bicarbonate (30 mL) followed by water (30 mL) and dried over sodium sulfate. The crude product was purified by flash chromatography (silica gel, 80:20 hexane-ethyl acetate) to give 200 mg (49%) of 2-(4'-*N*-*tert*-butyloxycarbonyl-methylaminophenyl)-6-bromobenzothiazole as a pale yellow solid; mp = 144.5-145.2 °C; $^1H$ NMR (300 MHz, $CDCl_3$) δ 8.02-8.00 (m, 3H), 7.89 (d, *J* = 8.4 Hz, 1H), 7.57 (d, *J* = 8.7 Hz, 1H), 7.40 (d, *J* = 8.1 Hz, 1H), 3.30 (s, 3H), 1.47 (s, 9H); $^{13}C$ NMR (75 MHz, $CDCl_3$) δ 167.88, 154.21, 153.01, 146.48, 136.63, 129.81, 129.57, 127.72, 125.21, 124.14, 118.60, 81.01, 67.95, 36.93, 28.29; MS (FAB) *m/z* 421 ($M^+$ + H, $^{81}Br$), 419 ($M^+$ + H, $^{79}Br$); Anal. ($C_{19}H_{19}BrN_2O_2S$) calcd: C, 54.42; H, 4.57; N, 6.68; S, 7.65. found: C, 54.34; H, 4.56; N, 6.78; S, 7.62.

(Step 3): Preparation of 2-(4'-*N*-*tert*-Butyloxycarbonyl-methylaminophenyl)-6-tributylstannylbenzothiazole

2-(4'-*N*-*tert*-Butyloxycarbonyl-methylaminophenyl)-6-tributylstannylbenzothiazole was used as a starting material instead of 2-(4'-nitrophenyl)-6-bromobenzothiazole.

A yellow oil; $^1H$ NMR (400 MHz, $CDCl_3$) δ 8.06-7.98 (m, 4H), 7.56 (d, *J* = 8.0 Hz, 1H), 7.38 (d, *J* = 8.8 Hz, 2H), 3.32 (s, 3H), 1.59-1.53 (m, 6H), 1.40-1.31 (m, 6H), 1.14-1.10 (m, 6H), 0.94-0.88 (m, 9H); $^{13}C$ NMR (75 MHz, $CDCl_3$) δ 166.81, 154.31, 154.02, 146.11, 139.05, 134.67, 133.78, 130.30, 129.04, 127.72, 125.24, 122.45, 36.98, 30.29, 29.07, 28.30, 27.36, 13.67, 9.82; MS (FAB) *m/z* 631 ($M^+$ + H); Anal. ($C_{31}H_{46}N_2O_2SSn$) calcd: C, 59.15; H, 7.37; N, 4.45; S, 5.09. found: C, 59.13; H, 7.34; N, 4.45; S, 5.04.

<Example 1> Preparation of 2-(4'-Aminophenyl)-6-[$^{18}F$]fluorobenzothiazole

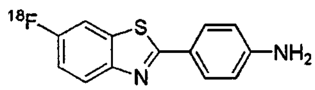

[$^{18}F$]Fluoride was produced in a cyclotron by the $^{18}O(p,n)^{18}F$ reaction. A volume of 100-200 μL [$^{18}F$]fluoride (18.5-370 MBq) in water was added to a vacutainer containing n-$Bu_4NHCO_3$ (40% aq. 2.12 μL, 2.76μmol). The azeotropic distillations were carried out each time with 200 μL aliquots of $CH_3CN$ at 85 °C under a stream of nitrogen. 2-(4'-Nitrophenyl)-6-iodophenyl(phenyl)benzothiazole iodonium tosylate (2 mg, 3.3 μmol) in acetonitrile (300 μL, adding 10 μL of $H_2O$ and 1 mg of TEMPO) was added to the dried tetrabuthylammonium fluoride salts in the reaction vial and reacted in the microwave equipment with 100W (180 sec). After the reaction, the vial was cooled in an ice bath and the solvent was removed under a gentle stream of nitrogen at 80 °C. The crude reaction mixture was diluted with 2 mL of ethanol-tetrahydrofuran-ethyl acetate (5:47.5:47.5, v/v), loaded into silica Sep-Pak and washed with 2 mL of the same solution again. The obtained solution was removed under a gentle stream of nitrogen and added tin(II) chloride (3.35 mg, 13 μmol) and EtOAc (200 μL). The mixture was heated at 80 °C for 10 min. The solvent was removed with a gentle stream of nitrogen. The reaction mixture was purified by HPLC at a flow 3 mL/min using a 30:7:63 mixture of 50 mM $(NH_4)H_2PO_4$-tetrahydrofuran-acetonitrile, and [$^{18}F$]**1** was eluted at 9.3 min. Radiotracer [$^{18}F$]**1** collected from HPLC was purified with Sep Pak cartridge with the help water (12 mL) and ethanol (1 mL), respectively. After the ethanol was evaporated, a target radiotracer was used for biological study. For the identification of the radio-product, the collected HPLC fraction was matched with the cold compound. Specific activity at the end of synthesis was calculated by relating radioactivity to the mass associated with the UV absorbance (254 nm) peak of cold compound. Specific radioactivity of

2-(4'-Aminophenyl)-6-[$^{18}$F]fluorobenzothiazole (42 GBq/μmol) was obtained after purification on analytic HPLC column.

<Example 2> Preparation of 2-(4'-*N*-Methylaminophenyl)-6-[$^{18}$F]fluorobenzothiazole

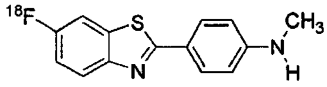

The preparation of [$^{18}$F]fluoride and the azeotropic distillations were carried out according to Example 1.

2-(4'-*N*-*tert*-Butyloxycarbonyl-methylaminophenyl)-6-iodophenyl(phenyl)benzothiazole iodonium tosylate (2 mg, 2.9 μmol) in acetonitrile (300 μL, adding 10 μL of $H_2O$ and 1 mg of TEMPO) was added to the dried tetrabuthylammonium fluoride salts in the reaction vial and reacted in the microwave equipment with 100W (180 sec). The vial was cooled in an ice bath and the solvent was removed under a gentle stream of nitrogen at 80 °C. 3 N HCl in ethyl acetate (3:1 ethyl acetate-conc. HCl, v/v, 250 μL) was added to the crude reaction mixture and reacted at 75 °C in oil bath for 10 minutes. After the reaction, the vial was cooled in an ice bath and the solvent was removed under a gentle steam of nitrogen at 75 °C. The reaction mixture was purified by HPLC at a flow 3 mL/min using a 45:55 mixture of $H_2O$-acetonitrile, and the product was eluted at 17.5 min. Radiotracer, collected from HPLC, was purified with Sep Pak cartridge with the help water (12 mL) and ethanol (1 mL), respectively. After the ethanol was evaporated, a target radiotracer was used for biological study. For the identification of the radio-product, the collected HPLC fraction was matched with the cold compound. Specific radioactivity of 2-(4'-*N*-Methylaminophenyl)-6-[$^{18}$F]fluorobenzothiazole (59 GBq/μmol) was obtained after purification on analytic HPLC column.

<Example 3> Preparation of 2-(4'-*N*-Dimethylaminophenyl)-6-[$^{18}$F]fluorobenzothiazole

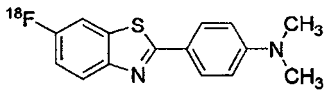

The preparation of [$^{18}$F]fluoride and the azeotropic distillations were carried out according to Example 1.

2-(4'-Dimethylaminophenyl)-6-iodophenyl(phenyl)benzothiazole iodonium tosylate (2 mg, 3.3 μmol) in acetonitrile (300 μL, adding 10 μL of $H_2O$ and 1 mg of TEMPO) was added to the dried tetrabuthylammonium fluoride salts in the reaction vial and reacted in the microwave equipment with 100W (180 sec). The vial was cooled in an ice bath and the solvent was removed under a gentle stream of nitrogen at 80 °C. The reaction mixture was purified by HPLC at a flow 3 mL/min using a 40:3:57 mixture of 50 mM $(NH_4)H_2PO_4$-tetrahydrofuran-acetonitrile, and the product was eluted at 15.7 min. Radiotracer, collected from HPLC, was purified with Sep Pak cartridge with the help water (12 mL) and ethanol (1 mL), respectively. After the ethanol was evaporated, a target radiotracer was used for biological study. For the identification of the radio-product, the collected HPLC fraction was matched with the cold compound. Specific radioactivity of [$^{18}$F]**3** (52 GBq/μmol) was obtained after purification on analytic HPLC column.

<Example 4 (not within the claims)> Preparation of 2-(4'-aminophenyl)-6-fluorobenzothiazole

(Step 1) Preparation of 2-(4'-Nitrophenyl)-6-fluorobenzothiazole

To a solution of 2-amino-5-fluorobenzenethiol (300 mg, 2.09 mmol) in dimethylsulfoxide (3 mL) was added 4-nitrobenzaldehyde (315 mg, 2.09 mmol). The reaction mixture was heated at 180 °C for 30 min. At the end of the reaction, the reaction mixture was cooled to room temperature and poured into ice-water (6 mL). The precipitate was filtered under reduced pressure. The filtrate was purified by recrystallization from tetrahydrofuran (5 mL)-methanol (150 mL) to

give 325 mg of 2-(4'-nitrophenyl)-6-fluorobenzothiazole (57%) as a yellow solid; mp = 201.2-201.5 °C; $^1H$ NMR (300 MHz, $CDCl_3$) δ 8.34 (d, *J* = 8.7 Hz, 2H), 8.21 (d, *J* = 8.7 Hz, 2H), 8.09-8.04 (m, 1H), 7.62 (dd, *J* = 8.1 2.4 Hz, 1H), 7.31-7.24 (m, 1H); $^{13}C$ NMR (75 MHz, $CDCl_3$) δ 162.00 (d, *J* = 246.6 Hz), 150.74 (d, *J* = 1.8 Hz), 149.02, 138.83, 136.50 (d, *J* = 11.2 Hz), 128.09, 124.97 (d, *J* = 9.2 Hz), 124.33, 115.78 (d, *J* = 24.8 Hz), 108.02 (d, *J* = 26.6 Hz); MS (EI) *m/z* 274 ($M^+$); Anal. ($C_{13}H_7FN_2O_2S$) calcd: C, 56.93; H, 2.57; N, 10.21; S, 11.69. found: C, 56.98; H, 2.60; N, 10.31; S, 11.69.

(Step 2) Preparation of 2-(4'-aminophenyl)-6-fluorobenzothiazole

To a solution of 2-(4'-nitrophenyl)-6-fluorobenzenethiol (1.6 g, 5.84 mmol) in ethanol (50 mL) was added tin(II) chloride e (4.92 mg, 26.3 mmol). The reaction mixture was heated at 100 °C under nitrogen gas for 1 h. Ethanol was removed by evaporator, and the residue was dissolved in ethyl acetate (100 mL), the organic solution was washed with saturated sodium bicarbonate (30 mL) followed by water (30 mL) and dried over sodium sulfate. The crude product was purified by flash choromatography (silica gel, 70:30 hexane-ethyl acetate) to give 1.05 g (74%) of 2-(4'-aminophenyl)-6-fluorobenzothiazole as a yellow solid; mp = 201.2-201.5 °C; Analytical HPLC: reverse phase (60:40 acetonitrile-$H_2O$) *k*' = 0.46, purity 98.37%; normal phase (10:90 5% IPA in dichloromethane-hexane) *k*' = 5.39, purity 98.14%; $^1H$ NMR (300 MHz, $CDCl_3$) δ 7.93-7.89 (m, 1H), 7.85 (d, *J* = 8.4 Hz, 2H), 7.52 (dd, *J* = 8.1, 2.4 Hz, 1H), 7.20-7.13 (m, 1H), 6.72 (d, *J* = 8.4 Hz, 2H), 4.01 (brs, 2H, $NH_2$);$^{13}C$ NMR (75 MHz, $CDCl_3$) δ 168.21 (d, *J* = 3.8 Hz), 160.06 (d, *J* = 242.9 Hz), 150.87 (d, *J* = 1.8 Hz), 149.26, 135.52 (d, *J* = 11.1 Hz), 129.01, 123.67, 123.22 (d, *J* = 9.3 Hz), 114.76, 114.46 (d, *J* = 24.0 Hz), 107.70 (d, *J* = 26.6 Hz); MS (CI) *m/z* 245 ($M^+$ + H); Anal. ($C_{13}H_9FN_2S$) calcd: C, 63.92; H, 3.71; N, 11.47; S, 13.13. found: C, 63.82; H, 3.71; N, 11.44; S, 13.24.

<Example 5 (not within the claims)> Preparation of 2-(4'-*N*-methylaminophenyl)-6-fluorobenzothiazole

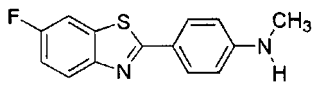

To a solution of 2-(4'-aminophenyl)-6-fluorobenzenethiol (300 mg, 1.23 mmol) in methanol (15 mL) was added formaldehyde (300 L, 3.68 mmol). The reaction mixture was refluxed for 2 h. Methanol was removed by evaporator and vacuum. The residue was dissolved in methano (100 mL) again, and was added sodium cyanoborohyde (310 mg, 4.92 mmol) and acetic acid (6 L, pH 6) slowly. The reaction mixture was stirred at room temperature for 1.5 h. Methanol was removed by evaporator and the residue was dissolved in ethyl acetate (50 mL), the organic solution was washed with saturated sodium bicarbonate (30 mL) followed by saline (30 mL) and dried over sodium sulfate. The crude product was purified by flash chromatography (silica gel, 70:30 hexane-ethyl acetate) to give 111 mg (35%) of 2-(4'-methylaminophenyl)-6-fluorobenzothiazole as a yellow solid; mp = 153.5-154.5 °C; Analytical HPLC: reverse phase (60:40 acetonitrile-$H_2O$) *k*' = 4.38, purity 99.67%; normal phase (10:90 5% IPA in dichloromethane-hexane) *k*' = 2.25, purity 98.58%; $^1H$ NMR (300 MHz, $CDCl_3$) δ 7.92-7.86 (m, 3H), 7.51 (dd, *J* = 8.1, 2.4 Hz, 1H), 7.20-7.12 (m, 1H), 6.63 (d, *J* = 8.4 Hz, 2H), 4.14 (brs, 1H, NH), 2.90 (s, 3H); $^{13}C$ NMR (75 MHz, $CDCl_3$) δ 168.49 (d, *J* = 3.1 Hz), 159. 96 (d, *J* = 242.3 Hz), 151.57, 150.97, 135.46 (d, *J* = 11.1 Hz), 128.95, 123.02 (d, *J* = 9.3 Hz), 122.25, 114.34 (d, *J* = 24.8 Hz), 112.01, 107.64 (d, *J* = 26.6 Hz), 30.26; MS (CI) *m/z* 259 ($M^+$ + H); Anal. ($C_{14}H_{11}FN_2S$) calcd: C, 65.10; H, 4.29; N, 10.84; S, 12.41. found: C, 65.13; H, 4.30; N, 10.86; S, 12.43.

<Example 6 (not within the claims)> Preparation of 2-(4'-Dimethylaminophenyl)-6-fluorobenzothiazole

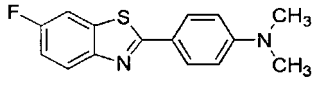

A dimethylation was carried out on the amine in 4' position in Example 5, followed by column chromatography to obtain a target compound.

A yellow solid; mp = 204.4-205.9 °C; Analytical HPLC: reverse phase (70:30 acetonitrile-$H_2O$) *k*' = 2.01, purity 99.8%; normal phase (10:90, 5% IPA in dichloromethane-hexane) *k*' = 0.06, purity 99.11%; $^1H$ NMR (300 MHz, $CDCl_3$) δ 7.92-7.87 (m, 3H), 7.50 (dd, *J* = 8.1, 2.4 Hz, 1H), 7.19-7.12 (m, 1H), 6.73 (d, *J* = 9.0 Hz, 2H), 3.05 (s, 6H); $^{13}C$ NMR (75 MHz, $CDCl_3$) δ 168.51 (d, *J* = 3.2 Hz), 159.92 (d, *J* = 242.3 Hz), 15-2.16, 151.04 (d, *J* = 1.88 Hz), 135.47 (d, *J* = 11.2

Hz), 128.72, 122.95 (d, *J* = 9.3 Hz), 121.12, 114.29 (d, *J* = 24.1 Hz), 111.67, 107.61 (d, *J* = 26.6 Hz), 40.11; MS (CI) *m/z* 273 ($M^+$ + H); Anal. ($C_{15}H_{13}FN_2S$) calcd: C, 66.15; H, 4.81; N, 10.29; S, 11.77. found: C, 66.13; H, 4.85; N, 10.27; S, 11.77.

<Example 7 (not within the claims)> Preparation of 6-fluorine-2-(4'-N-phenylacetamide)benzothiazole

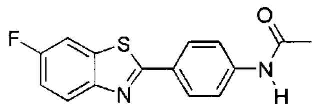

The compound 6-fluorine-2-(4'-aminophenyl)benzothiazole (20 mg, 0.08 mmol) prepared in Example 1 was dissolved in acetonitrile (2 mℓ). A mixture of acetyl chloride (10 μℓ, 0.16 mmol) and triethylamine (1 mℓ) was slowly dripped into the resulting solution at 0°C and then the mixture was stirred at 90 °C for 1 hour. After the mixture was cooled at room temperature, the remaining acetonitrile was removed at reduced pressure, water was added into the mixture, and an extraction was performed with methylene chloride (20 mℓ×3) as an organic solvent, followed by column chromatography to obtain a target compound.

$^1$H NMR (400 MHz, DMSO-$d_6$) δ 8.23 (d, J = 2.0 Hz, 1H), 7.75 (d, J = 8.8 Hz, 1H), 7.69 (d, J = 5.2 Hz, 2H), 7.53 (dd, J = 8.0 Hz, J = 2.0 Hz, 1H), 6.61 (d, J = 8.8 Hz, J = 2.0 Hz, 2H), 5.01 (s, H, NH2), 2.03 (s, $CH_3$); MS (EI) m/z 347 ($M^+$).

<Example 8 (not within the claims)> Preparation of 6-fluorine-2-(4'-N-(2"-(2"-methoxyethoxy)acetamidephenyl))benzothiazole

3-(2-methoxyethoxy)propionic acid (0.16 mℓ, 0.8 mmol) was dissolved in methylene chloride (1 mℓ), thionyl chloride ($SOCl_2$, 1.5 mℓ) was slowly dripped into the resulting solution at 0°C and then the mixture was stirred under reflux at 60 °C in an oil bath for 1 hour. The solvent and thionyl chloride in the mixture were removed, the resulting mixture was dissolved again in acetonitrile (2.5 mℓ) as a solvent, a mixture of 6-fluorine-2-(4'-aminophenyl)benzothiazole (100 mg, 0.4 mmol) and triethylamine (1 mℓ) was introduced into the solution, and the resulting mixture was stirred at 90 °C for 30 minutes. After the mixture was cooled at room temperature, the remaining acetonitrile was removed at reduced pressure, water (100 mℓ) was added into the mixture, and an extraction was performed with methylene chloride (100 mℓ× 3) as an organic solvent, followed by column chromatography to obtain a target compound.

$^1$H NMR (300 MHz, $CDCl_3$) δ 9.12 (s, 1H), 8.59-8.02 (m, 2H), 8.01-7.97 (m, 1H), 7.77-7.73 (m, 2H), 7.57 (dd, J = 6.00 Hz, J = 1.80 Hz, 1H), 7.24-7.19 (m, 1H), 2.10 (s, 2H), 3.80-3.78 (m, 2H), 3.65-3.63 (m, 2H), 3.50 (s, 3H); $^{13}$C NMR (400 MHz, $CDCl_3$) δ 168.59, 158.73, 150.77, 150.74, 140.08, 135.95, 135.80, 129.17, 128.26, 123.91, 123.78, 119.70, 115.01, 112.43, 107.97, 107.61, 71.40, 71.31, 70.42, 59.07; MS (CI) m/z 362 ($M^+$ +1).

<Example 9 (not within the claims)> Preparation of 6-fluorine-2-(4'-N-(2"-(2"-(2"-methoxyethoxy)ethoxy)acetamidephenyl)benzothiazole

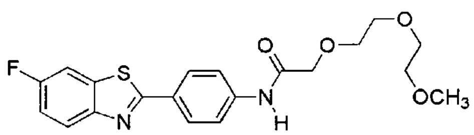

A target compound was obtained in the same way as in Example 8 except that 3-[2-(2-methoxyethoxy)ethoxy] propionic acid was used instead of 3-(2-methoxyethoxy)propionic acid.

$^1$H NMR (300 MHz, $CDCl_3$) δ 8.98 (s, 1H), 8.04-8.02 (m, 2H) 8.01-7.97 (m, 1H), 7.89 (dd, , J = 5. 10 Hz, J = 1.50 Hz, 1H), 7.57 (dd, , J = 5.10 Hz, J = 6.30 Hz, 1H), 7.24-7.19 (m, 1H), 4.14 (s, 2H), 3.81-3.79 (m, 2H), 3.75-3.73 (m, 4H), 3.61-3.59 (m, 2H) 3.39 (s, 3H); $^{13}$C NMR (400 MHz, $CDCl_3$) δ 168.54, 167.28, 161.99, 158.74, 156.24, 150.78, 150.77, 140.04, 135.96, 135.82, 129.22, 128.19, 123.92, 123.79, 120.07, 115.02, 114.70, 107.98, 107.62, 71.73, 71.23, 70.72, 70.42, 70.08, 59.01; MS (CI) m/z 405 ($M^+$ +1).

<Example 10 (not within the claims)> Preparation of 6-fluorine-2-(4'-N-(2"-(2"-methoxyethoxy)ethoxyaminophenyl))benzothiazole

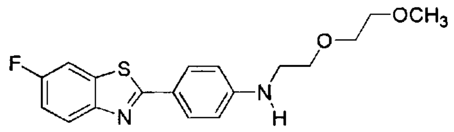

6-fluorine-2-(4'-N-(2"-(2"-methoxyethoxy)acetamidephenyl)benzothiazole (10 mg, 0.03 mmol), the compound in Example 8, was dissolved in anhydrous tetrahydrofuran (THF) (2 mℓ). 1 M lithium aluminum hydride (LAH)(0.1 mℓ, 0.15 mmol) dissolved in ether was slowly dripped into the resulting solution at °C and then the mixture was stirred for 1 hour. After the mixture was cooled at room temperature, the remaining solvent was removed under reduced pressure, water (20 mℓ) was added into the resulting mixture, and an extraction was performed with methylene chloride (30 mℓ×3) as an organic solvent, followed by column chromatography to obtain a target compound.

$^{1}$H NMR (300 MHz, $CDCl_3$) δ 7.91 (d, J = 4.00 Hz, 1H), 7.87 (d, J = 8.00 Hz, 2H), 7.52 (dd, J = 8.00 Hz, J = 4.00 Hz, 1H), 7.17-7.13 (m, 1H), 6.66 (d, J = 8.00 Hz, 2H), 4.14-4.09 (m, 2H), 3.75-3.73 (m, 2H), 3.67-3.65 (m, 2H), 3.58-3.56 (m, 2H), 3.41 (s, 3H); MS (EI) m/z 346 ($M^+$).

<Example 11> Preparation of 2-(4'-Nitrophenyl)-6-iodophenyl(phenyl)benzothiazole iodonium tosylate

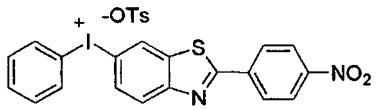

To a solution of Koser's reagent (69.5 mg, 0.17 mmol) in dichloromethane (10 mL) was added 2-(4'-nitrophenyl)-6-tributylstannylbenzothiazole (95 mg, 0.17 mmol) obtained in Preparation Example 1 under argon atmosphere. The reaction mixture was stirred at room temperature under argon atmosphere for 12 h. The solvent was evaporated using a stream of nitrogen. The crude mixture was dissolved a small amount of methanol (1.5 mL) and transferred to the centrifuge tube to which was added excess diethyl ether (20 mL). After centrifuging, the collected oil was dried in vacuo to give 40 mg (38%) of 2-(4'-nitrophenyl)-6-iodophenyl(phenyl)benzothiazole iodonium tosylate as a yellow solid: mp = 216.6-218.5 °C; $^{1}$H NMR (400 MHz, MeOH-$d_4$) δ 9.02 (d, $J$ = 1.6 Hz, 1H), 8.43-8.37 (m, 5H), 8.31 (dd, $J$ = 8.0, 2.0 Hz, 1H), 8.24-8.18 (m, 3H), 7.68 (d, $J$ = 8.0 Hz, 2H), 7.54 (t, $J$ = 8.0 Hz, 2H), 7.21 (d, $J$ = 8.0 Hz, 2H), 2.34 (s, 3H); $^{13}$C NMR (100 MHz, MeOH-$d_4$) δ 168.30, 154.33, 148.43, 140.77, 138.88, 136.77, 136.37, 133.74, 131.55, 131.04, 130.50, 128.69, 127.21, 127.03, 125.00, 124.18, 122.77, 113.89, 109.20, 18.54; MS (FAB) $m/z$ 459 ($M^+$ + H -OTs); HRMS calcd for $C_{19}H_{12}IN_2O_2S$ 458.9664, found 458.9671.

<Example 12> Preparation of 2-(4'-*N-tert*-Butyloxycarbonyl-methylaminophenyl)-6-iodophenyl(phenyl)benzothiazole iodonium tosylate

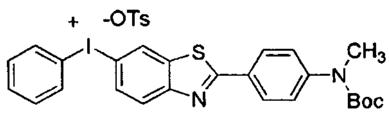

A target compound was obtained in the same way as in Example 11, except that the 2-(4'-N-methyl-N-t-butyloxycarbonylaminophenyl)-6-tributylstannylbenzothiazole prepared in Preparation Example 3 was used as a starting material instead of 2-(4'-nitrophenyl)-6-tributylstannylbenzothiazole

A yellow solid; mp 156.2-156.9; $^{1}$H NMR (400 MHz, MeOH-$d_4$) δ 8.92 (d, $J$ = 1.6 Hz, 1H), 8.26-8.20 (m, 3H), 8.13-8.06 (m, 3H), 7.70-7.63 (m, 3H), 7.56-7.47 (m, 4H), 7.19 (d, $J$ = 8.0 Hz, 2H), 3.32 (s, 3H), 1.47 (s, 9H); $^{13}$C NMR (100 MHz, MeOH-$d_4$) δ 173.26, 157.30, 155.92, 148.67, 143.56, 141.63, 139.11, 136.40, 134.08, 133.74, 133.23, 131.13,

130.89, 130.51, 129.78, 129.28, 127.67, 126.99, 126.95, 116.64, 110.86, 82.46, 37.45, 28.51, 21.30; MS (FAB) *m/z* 543 ($M^+$ + H-OTs) ; HRMS calcd for $C_{25}H_{24}IN_2O_2S$ 543.0603, found 543.0599.

<Example 13> Preparation of 2-(4'-Dimethylaminophenyl)-6-iodophenyl(phenyl)benzothiazole iodonium tosylate

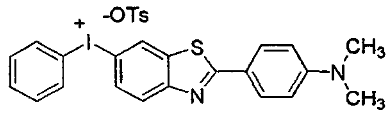

2-(4'-N,N-dimethylaminophenyl)-6-tributylstannylbenzothiazole was used as a starting material instead of 2-(4'-nitrophenyl)-6-tributylstannylbenzothiazole to obtain a target compound.

A yellow solid; mp = 172.0-173.8 °C; $^1H$ NMR (400 MHz, MeOH-$d_4$) δ 8.81 (d, *J* = 2.0 Hz, 1H), 8.21-8.17 (m, 3H), 7.95-7.92 (m, 3H) 7.70-7.67 (m, 3H), 7.53 (t, *J* = 7.8 Hz, 2H), 7.20 (d, *J* = 8.0 Hz, 2H), 6.82 (d, *J* = 8.8 Hz, 2H), 3.08 (s, 6H), 2.34 (s, 3H); $^{13}C$ NMR (100 MHz, MeOH-$d_4$) δ 156.55, 153.76, 150.39, 140.98, 140.48, 135.07, 133.61, 132.78, 132.48, 131,99, 129.42, 129.24, 128.60, 125.75, 124.42, 116.27, 111.77, 108.07, 81.18, 39.10, 16.16; MS (FAB) *m/z* 457 ($M^+$ + H -OTs); HRMS calcd for $C_{21}H_{18}IN_2S$ 457.0235, found 457.0246.

<Evaluation of biological activity of [18F]fluorine benzothiazole derivatives>

Measurement of partition coefficient

The [$^{18}$F]fluorine bezothiazole derivatives according to the present invention should cross the BBB in brain for *in vivo* binding to beta-amyloid plaques. Then, the lipophilicity of the compound is a significantly important factor, and it is possible to determine the cerebral uptake and release degree initially with the factor. For this purpose, the distribution ratio of octanol to water buffer of the compound to be measured may be measured to determine the relative lipophilicity of each compound. The experimental method is as follows: A mixture of 5 mℓ of 1-octanol and 5 mℓ of 1 M PBS buffer was prepared, each compound (0.37 MBq) in Examples 1 to 3 was dissolved in ethanol (0.1 mℓ), and the resulting solution was introduced into the mixture. The mixture was vortexed at room temperature for 5 minutes and then was centrifuged at 1000 rpm for 5 minutes. 4 mℓ was withdrawn from the octanol layer of the solution, introduced into a new tube, and another 4 mℓ of 1 M PBS buffer was introduced into the tube. The mixture was vortexed at room temperature for 5 minutes and then centrifuged at 1000 rpm for 5 minutes. 50 μℓ was withdrawn from the octanol layer and introduced into a test tube, and 500 μℓ was withdrawn from the 1 M PBS buffer and introduced into the test tube. 3 mℓ was withdrawn from the octanol layer and introduced into another test tube, and another 3 mℓ of 1 M PBS buffer was added into the test tube. The mixture was vortexed at room temperature for 5 minutes and then centrifuged at 1000 rpm for 5 minutes. 50 μℓ was withdrawn from the octanol layer and introduced into a test tube, and 500 μℓ was withdrawn from the 1 M PBS buffer and introduced into the test tube. 2 mℓ was withdrawn from the octanol layer and introduced into another test tube, and another 2 mℓ of the PBS buffer. The mixture was vortexed at room temperature for 5 minutes and then centrifuged at 1000 rpm for 5 minutes. 50 μℓ was withdrawn from the octanol layer and introduced into a test tube, and 500 μℓ was withdrawn from the 1 M PBS buffer and introduced into the test tube. The process was repeated three times as in the above way. Each of the octanol and 1 M PBS buffer in the test tube was measured using a gamma counter, and the results were substituted into the following Formula 1 to obtain a log P value.

<Formula 1>

$$\log P = [octanol]/[buffer]$$

The log P vales of three compounds (Examples 1, 2, and 3) among the [$^{18}$F]fluorine benzothiazole derivatives were measured and the values are as follows.

[Table 2]

| Compound in Examples | Log P value |
|---|---|
| Example 1 | 3.93 |

(continued)

| Compound in Examples | Log P value |
|---|---|
| Example 2 | 3.11 |
| Example 3 | 4.33 |

Referring to Table 2, a log P value (partition coefficient) in Example 1 where a methyl was not substituted into an amine group was smaller than that in Example 3 where two methyl groups were substituted, and it was determined that the value was similar to a value expected from the structure of the derivative. In addition, the log P value in Example 2 was 3.11, from which it may be deduced that the compound in Example 2 was probably the fastest in release after cerebral uptake from among the three compounds.

<Experimental Example> Measurement of binding affinity to beta-amyloid plaques

1.1. Preparation of beta-amyloid fibrils

In order to measure a binding force to the beta-amyloid plaques of the fluorinated benzothiazole derivatives according to the present invention, fibrils were preparaed through the following experiment.

A fibril solution, in which 1 mg of each of beta-amyloid plaque peptides ($A\beta_{1-40}$ and $A\beta_{1-42}$) to be used for analysis was dissolved in 1.155 mℓ of ethylenediaminetetraacetic acid disodium salt ($Na_2EDTA$) solution, was introduced into 20 mM PBS buffer (pH 7.4), a sonication was performed for 30 minutes, and then the mixture was incubated with stirring for 3 days at 30°C. The thus-obtained solution was high speed centrifuged (28,000 g for 15 minutes) at 4°C, and the supernatant was collected. The precipitate was washed twice with 100 μℓ of a mixture solution of 1 mM ethylenediaminetetraacetic acid disodium salt and 10 mM PBS buffer. The precipitate was resuspended in 2.310 mℓ of 1 mM ethylenediaminetetraacetic acid disodium salt and 10 mM PBS buffer, and the suspended sold produced was divided into equal aliquots of 30 μℓ and kept at -80°C. The concentration of fibrils in the aliquots of 30 μℓ was 100 μM, respectively.

1.2. Measurement of binding affinity

The radioactive ligand to be used for binding analysis was 2-(3-[125I]iodo-4'-N-methylaminophenyl)benzothiazole with the specific radioactivity of $8.05 \times 10^{16}$ Bq/mol, and $K_d$ values for the known Aβ1-40 and Aβ1-40 were 2.30 ± 0.33 nM and 0.44 ± 0.25 nM. Each tube to be used in measurement was filled with 860 μℓ of 10% ethanol physiological saline solution, and the compounds at $10^{-4}$ to $10^{-9}$ M were dissolved in phosphate buffered saline (PBS, pH 7.4) containing 10% ethanol to form solutions. Each of 40 μℓ of the solutions was introduced into the tube, respectively. 50 μℓ of $[^{125}I]$ TZDM (32 kBq/mℓ) and 50 μℓ of beta-amyloid fibrils dissolved in the prepared PBS (30 μℓ of aliquots prepared was diluted 200-fold with PBS solution and the final concentration was 20 nM when 50 μℓ was used) were introduced into the tube. A mixed solution with a total volume of 1000 μℓ was incubated at room temperature for 3 hours, and then a Whatman GF/B filter was used to separate the solution into unbound radioactive materials and bound radioactive materials. Then, each tube was washed three times with 3 mℓ of 10% ethanol. The radioactivity was measured using an automatic gamma counter.

The binding affinity (Ki) to beta-amyloid fibrils of the compounds of the present invention was measured, and the results were shown in the following Table 3.

[Table 3]

| Compound | Aβ1-40 | Aβ1-42 |
|---|---|---|
| | Ki (nM) | Ki (nM) |
| Example 4 | 52.4 | 22.2 |
| Example 5 | 13.3 | 7.55 |
| Example 6 | 7.9 | 1.86 |

As indicated in Table 3, the compounds obtained in Examples 4, 5, and 6 of the present invention showed binding affinities similar to that of $[^{11}C]$PIB($A\beta_{1-40}$, 4.3), a representative benzothiazole-series beta-amyloid plaque-imaging radiopharmaceutical, and particularly, the compounds in Example 6 showed the best binding affinity among compounds so far developed.

1.3 Evaluation of in vivo cerebral uptake and release degree in a normal mouse

6 week-old ICR mice were used for experiments on cerebral uptake and release degree of three 6-[18F]fluorine-2-allylbenzothiazole derivatives (compounds in Examples 1 to 3) in a mouse over time. Each of the compounds in Examples 1 to 3 was dissolved in a physiological saline solution containing 5% ethanol, and each of 200 $\mu\ell$ (100 $\mu$Ci) of the solutions was injected through the tail vein of the mouse. 2, 30, and 60 minutes were selected as in-vivo retention time periods, and 4 to 7 mice were used in each time period. After the mice were sacrificed by cervical dislocation in each time period, the brains were quickly removed, they were separated into three parts of cortex, cerebellum, and remnant on filter paper kept on ice, respectively, and blood was collected. The specimen thus-obtained was introduced into a glass tube, the weight was determined, and the radioactivity were measured using a gamma counter. Using the data obtained, the percent injected dose per gram of tissue (%ID/g) in each sample compared to radioactivity actually injected, and the value (%ID-kg/g) corrected for individual body weights of the mice used were calculated. The results were shown in the following Tables 4 to 6 and FIG. 1.

[Table 4]

| Example 1 | | | | | | |
|---|---|---|---|---|---|---|
| Tissue | %ID / g | | | %ID-kg / g | | |
| | 2 min | 30 min | 60 min | 2 min | 30 min | 60 min |
| Blood | 1.92 ±0.10 | 1.23 ±0.51 | 1.21 ±0.08 | 0.072 ±0.005 | 0.043 ±0.008 | 0.052 ±0.004 |
| Cortex | 5.86 ±0.34 | 1.79 ±0.29 | 0.73 ±0.10 | 0.151 ±0.008 | 0.047 ±0.006 | 0.019 ±0.003 |
| Cerebe llum | 5.83 ±0.18 | 2.19 ±0.33 | 1.02 ±0.15 | 0.150 ±0.006 | 0.058 ±0.007 | 0.027 ±0.004 |
| Remnan t | 6.41 ±0.19 | 2.86 ±0.39 | 1.37 ±0.14 | 0.165 ±0.007 | 0.075 ±0.008 | 0.036 ±0.005 |

[Table 5]

| Example 2 | | | | | | |
|---|---|---|---|---|---|---|
| Tissue | %ID / g | | | %ID-kg / g | | |
| | 2 min | 30 min | 60 min | 2 min | 30 min | 60 min |
| Blood | 2.08± 0.19 | 0.92± 0.15 | 1.28± 0.08 | 0.070± 0.007 | 0.032± 0.007 | 0.042± 0.006 |
| Cortex | 6.62± 0.33 | 1.20± 0.20 | 0.73± 0.10 | 0.222± 0.015 | 0.042± 0.010 | 0.024± 0.002 |
| Cerebellum | 6.24± 0.35 | 1.38± 0.23 | 0.98± 0.12 | 0.210± 0.017 | 0.048± 0.011 | 0.032± 0.003 |
| Remnant | 6.30± 0.46 | 1.91± 0.30 | 1.32± 0.15 | 0.212± 0.017 | 0.066± 0.014 | 0.044± 0.003 |

[Table 6]

| Example 3 | | | | | | |
|---|---|---|---|---|---|---|
| Tissue | %ID / g | | | %ID-kg / g | | |
| | 2 min | 30 min | 60 min | 2 min | 30 min | 60 min |
| Blood | 1.55± 0.08 | 1.04± 0.19 | 1.02± 0.06 | 0.040± 0.003 | 0.026± 0.005 | 0.021± 0.010 |
| Cortex | 4.39± 0.22 | 2.17± 0.26 | 1.61± 0.22 | 0.114± 0.004 | 0.054± 0.006 | 0.033± 0.017 |
| Cerebellum | 4.45±0.19 | 2.03±0.26 | 1.16±0.09 | 0.115±0.005 | 0.050±0.007 | 0.024±0.012 |
| Remnant | 4.26± 0.22 | 2.80± 0.39 | 2.01± 0.15 | 0.110± 0.006 | 0.069± 0.010 | 0.041± 0.020 |

From the results in Tables 4 to 6 and FIG. 1, the 2-minute cerebral uptake images of the compounds in Examples 1, 2, and 3 and their release degrees in 30 and 60 minutes showed that the compounds in Examples 1 and 2 had high uptakes in the cortexes with 5.86 ± 0.34 and 6.62 ± 0.33 at initial 2 minutes, and with 0.73 ± 0.10 and 0.73 ± 0.09 at 60 minutes. From the values into which the radioactivity remaining in the in vivo cortex per hour was calculated as a

percentage, it was confirmed that the compounds were very quickly released. From the pre-clinical results, it can be known that the derivatives of the present invention are quickly released in a normal mouse brain without beta-amyloid plaques in 1 hour and are a promising compound which enhances the quality of background imaging and provides an accuracy of diagnosis of Alzheimer's disease. It was shown that the compound in Example 3, which had high binding affinity to ex vivo beta-amyloid fibrils, had a relatively lower initial cerebral uptake and a slower 60-minute removal rate than those of the other compounds, due to the lipophilicity of the compound itself.

1.4. Evaluation of the brain imaging of normal male volunteers.

Each of the compounds (196 MBq) in Examples 2 and 3 of the present invention was dissolved in 6 mℓ of physiological saline solution including 5% ethanol, the resulting solution was intravenously injected into a normal volunteer (male, 37 years old, 78 kg), and Static Images of the brain from the injection to 2 hours were obtained through a Phillips Allegro PET scanner. The protocol of the brain imaging repeated emission and transmission as follows.

[2 minute emission + transmission] × four times

[5 minute emission + transmission] × four times

[10 minute emission + transmission] × three times

For attenuation correction, a Cs-137 radiation source prior to the administration was used to obtain a one-and-a half minute transmission scan per bed (18 cm). The transmission image and a 3D Row-Action Maximum-Likelihood (RAMLA) algorithm were used to reconstruct an attenuation corrected image. The 3D voxel size was 2.0 × 2.0 × 2.0 mm and the matrix size was 128 × 128 × 90 (FIGs. 2 and 3). The results of brain region to cerebellar ratio at 15 and 90 minutes were shown in Table 7.

[Table 7]

| Compound | Imaging time | Cinerea | posterior cingulate cortex | Frontal cortex | Occipital cortex | Cerebellum |
|---|---|---|---|---|---|---|
| Example 2 | 15 min | 0.45 | 0.89 | 0.92 | 1.05 | 1.00 |
| | 90 min | 0.83 | 0.87 | 0.96 | 0.95 | 1.00 |
| Example 3 | 15 min | 0.40 | 0.91 | 0.88 | 1.17 | 1.00 |
| | 90 min | 0.83 | 1.00 | 1.02 | 1.12 | 1.00 |

Referring to Table 7, cortices had ratios similar to that of the cerebellum over time, and the cinerea showed a gradually increasing ratio compared to that of the cerebellum. From these, it can be known that the wash-out of the cortices in Examples 2 and 3 of the present invention decreased similarly to that of the cerebellum. However, it can be known that the cinerea showed a slow wash-out. It can be known that this was the same result as the PIB images developed in the prior art.

From the FIGs. 2 to 4 showing release images from administration to 2 hours, it can be known that the derivatives according to the present invention were quickly released in 2 hours, and that these effects are even better especially in the compound in Example 2 than in the others.

## Claims

1. A compound represented by below Chemical Formula 1:

【Chemical Formula 1】

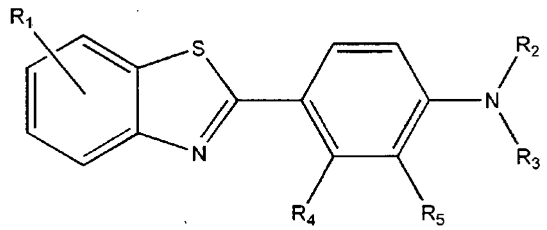

wherein,
$R_1$ is $^{18}F$, and $R_1$ is substituted into one in 4, 5, 6, and 7 positions of the benzothiazole ring;
$R_2$ is one selected from the group consisting of hydrogen, $C_1$-$C_4$ linear or branched alkyl, $C_1$-$C_4$ linear or branched alkylcarbonyl, 2-(2'-methoxy-(ethoxy)$_n$)$C_1$-$C_4$ linear or branched alkylcarbonyl, and 2-(2'-methoxy-(ethoxy)$_n$)$C_1$-$C_4$ linear or branched alkyl, wherein n is an integer of 1 to 5; $R_3$ is hydrogen, or $C_1$-$C_4$ linear or branched alkyl; and $R_4$ and $R_9$ are each independently hydrogen or hydroxy.

**2.** The compound according to claim 1, wherein $R_2$ is hydrogen, methyl, acetyl, 2-(2'-methoxy-(ethoxy)$_n$) acetyl, or 2-(2'-methoxy-(ethoxy)$_n$) ethyl, wherein n is an integer of 1 to 5; and $R_3$ is hydrogen or methyl.

**3.** The compound according to claim 1, wherein the compound is one selected from the group consisting of 1)6-[18F]fluoro-2-(4'-aminophenyl)benzothiazole; 2)6-[18F]fluoro-2-(4'-N-methylaminophenyl)benzothiazole; and 3)6-[18F]fluoro-2-(4'-N,N-dimethylaminophenyl)benzothiazole.

**4.** A method for preparing the compound according to claim 1, as indicated in the following Reaction Formula 1, wherein a mixture of [$^{18}$F]fluorine and tetrabutylammoniumcarbonate (TBA) is used and reacted with a compound of Chemical Formula 2 to label the $^{18}$F directly to the benzothiazole ring:

<Reaction Formula 1>

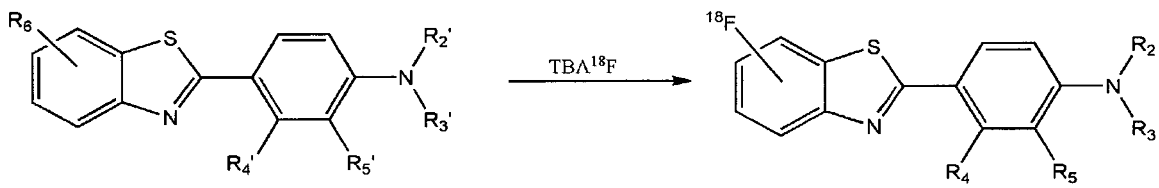


2 1a

wherein,
$R_2$, $R_3$, $R_4$, and $R_5$ are the same as defined for the compound of Chemical Formula 1 in claim 1 above;
$R_6$ is iodophenyltosylate (

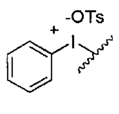

), 2-iodothiophenetosylate (

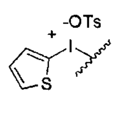

), or 2-iodoresinthiophenetosylate (

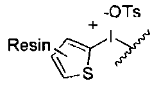

);

$R_{2'}$ is one selected from the group consisting of the $R_2$ substituents as defined for the compound of Chemical Formula 1 in claim 1, and oxygen,

$R_{3'}$ is one selected from the group consisting of the $R_3$ substituents as defined for the compound of Chemical Formula 1 in claim 1, t-butoxycarbonyl (Boc) and oxygen,

and when one of $R_{2'}$ and $R_{3'}$ is hydrogen, the other is also hydrogen, and when $R_{3'}$ is t-butoxycarbonyl (Boc), $R_3$ is hydrogen;

and

$R_{4'}$ and $R_{5'}$ are each independently one selected from the group consisting of hydrogen and methoxymethyl (MOM) ether;

and,

if necessary, carrying out a reduction, alkylation, deprotection and/or acylation reaction to produce said compound of Chemical Formula I.

**5.** The method according to claim 4, wherein introduction of $^{18}F$ is performed through a process, where a mixture of [$^{18}F$]fluorine and TBA is introduced into a vacutainer and nitrogen gas is blown at 75°C to 85°C into the container to dry the [$^{18}F$]fluorine (Step 1); and the dried [$^{18}F$]fluorine in Step 1 is transferred to a reaction vessel in which a starting material of Chemical Formula 2 as described in Reaction Formula 1 and 2,2,6,6-tetramethylpiperidine-N-oxyl (TEMPO) are dissolved in a acetonitrile/water solvent, followed by irradiation of microwave onto the reaction vessel (Step 2).

**6.** Benzothiazole precursors, represented by the following Chemical Formula 2, which are used in a reaction for introducing $^{18}F$ directly:

【Chemical Formula 2】

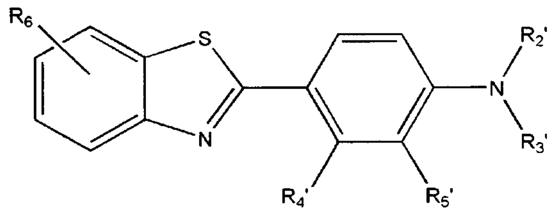

wherein, $R_6$, $R_{2'}$, $R_{3'}$, $R_{4'}$, and $R_{5'}$ are the same as defined in Reaction Formula 1 of claim 4.

**7.** Benzothiazole precursors according to claim 6, wherein the chemical compound of Chemical Formula 2 is one selected from the group consisting of 6-iodophenyl-2-(4'-nitrophenyl)benzothiazoleiodoniumtosylate; 6-iodophenyl-2-(4'-N-methyl(t-butyloxycarbonyl)aminophenyl)benzothiazoleiodoniumtosylate; and 6-iodophenyl-2-(4'-N,N-dimethylaminophenyl)benzothiazoleiodoniumtosylate.

**8.** A method for preparing benzothiazole precursors of claim 6 as indicated in the following Reaction Formula 3:

<Reaction Formula 3>

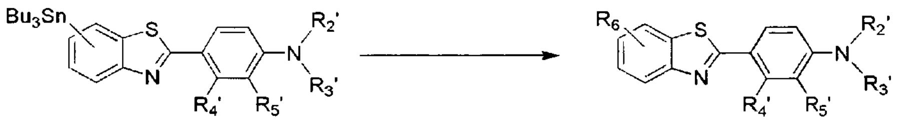

8 2

wherein, $R_6$, $R_{2'}$, $R_{3'}$, $R_{4'}$, and $R_5$, are the same as defined in Reaction Formula 1 of claim 4.

**9.** The method according to claim 8, wherein a reactant to introduce of a -$R_6$ group is hydroxytosyloxyiodobenzene (Koser's reagent),
2-hydroxytosyloxyiodothiophene or 2-hydroxytosyloxyiodothiophene bound to resin.

**10.** The method according to claim 8, wherein the reaction is performed by dissolving the reactant of claim 9 in acetonitrile solvent under inert gas atmosphere, dripping the compound (8) dissolved in methylene chloride at 0°C or less, and stirring the compound at room temperature for 12 to 15 hours.

**11.** An imaging agent for use in diagnosing Alzheimer's disease comprising the compound of claim 1.

**12.** The compound according to claim 1 for use in diagnosing presence and severity of Alzheimer's disease, wherein the compound is administered to the subject requiring a diagnosis of Alzheimer's disease, and direct visualization of beta-amyloid plaques is carried out using positron emission tomography through forming a bond of the compound with the beta-amyloid plaques.

**Patentansprüche**

**1.** Verbindung mit der nachfolgenden chemischen Formel 1:

[Chemische Formel 1]

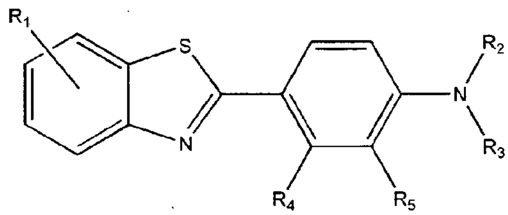

worin
$R_1$ gleich $^{18}F$ ist und $R_1$ an der 4-, 5-, 6- oder 7-Position des Benzothiazolringes substituiert ist;
$R_2$ ein Rest ist, der aus der Gruppe ausgewählt ist, bestehend aus Wasserstoff, linearem oder verzweigtem $C_1$-$C_4$-Alkyl, linearem oder verzweigtem $C_1$-$C_4$-Alkylcarbonyl, linearem oder verzweigtem 2-(2'-Methoxy-(ethoxy)$_n$)-$C_1$-$C_4$-alkylcarbonyl und linearem oder verzweigtem 2-(2'-Methoxy-(ethoxy)$_n$)-$C_1$-$C_4$-alkyl, wobei n eine ganze Zahl von 1 bis 5 ist; $R_3$ Wasserstoff oder lineares oder verzweigtes $C_1$-$C_4$-Alkyl ist; und
$R_4$ und $R_5$ jeweils unabhängig voneinander Wasserstoff oder Hydroxyl sind.

**2.** Verbindung nach Anspruch 1,
wobei $R_2$ Wasserstoff, Methyl, Acetyl, 2-(2'-Methoxy-(ethoxy )$_n$)-acetyl oder 2-(2'-Methoxy-(ethoxy)$_n$)-ethyl ist, wobei n eine ganze Zahl von 1 bis 5 ist, und $R_3$ Wasserstoff oder Methyl ist.

**3.** Verbindung nach Anspruch 1,
wobei die Verbindung eine solche ist, die aus der Gruppe ausgewählt ist, bestehend aus:

1) 6-[18F]Fluor-2-(4'-aminophenyl)benzothiazol;

2) 6-[18F]Fluor-2-(4'-N-methylaminophenyl)benzothiazol; und
3) 6-[18F]Fluor-2-(4'-N,N-dimetylaminophenyl)benzothiazol.

**4.** Verfahren zum Herstellen einer Verbindung nach Anspruch 1, wie es im folgenden Reaktionsschema 1 angegeben ist, wobei ein Gemisch von Fluor [$^{18}F$] und Tetrabutylammoniumcarbonat (TBA) verwendet und mit einer Verbindung der chemischen Formel 2 umgesetzt wird, um den Benzothiazolring direkt mit $^{18}F$ zu markieren:

<Reaktionsschema 1>

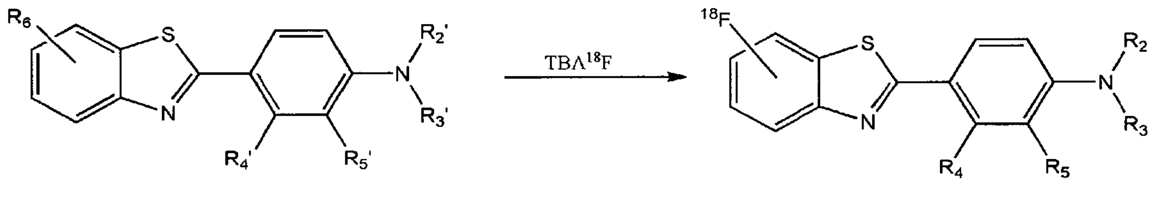


2 1a

worin
$R_2$, $R_3$, $R_4$ und $R_5$ wie vorstehend für die Verbindung mit der chemischen Formel 1 in Anspruch 1 angegeben sind;
$R_6$ Iodphenyltosylat (

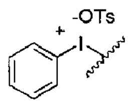

), 2-Iodthiophentosylat (

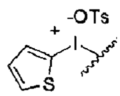

) oder
2-Iodharzthiophentosylat (

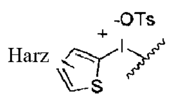

) ist;
$R_{2'}$ ein Rest ist, der aus der Gruppe ausgewählt ist, die aus den Substituenten $R_2$, wie sie für die Verbindung mit der chemischen Formel 1 in Anspruch 1 angegeben sind, und Sauerstoff besteht;
$R_{3'}$ ein Rest ist, der aus der Gruppe ausgewählt ist, die aus den Substituenten $R_3$, wie sie für die Verbindung mit der chemischen Formel 1 in Anspruch 1 angegeben sind, t-Butoxycarbonyl (Boc) und Sauerstoff besteht, und wenn einer der Reste $R_{2'}$ und $R_{3'}$ Wasserstoff ist, der andere ebenfalls Wasserstoff ist, und wenn $R_{3'}$ t-Butoxycarbonyl (Boc) ist, $R_3$ Wasserstoff ist;
und
$R_{4'}$ und $R_{5'}$ jeweils unabhängig voneinander ein Rest sind, der aus der Gruppe ausgewählt ist, die aus Wasserstoff und Methoxymethylether (MOM-ether) besteht; und
falls erforderlich eine Reduktion, Alkylierung, das Entfernen von Schutzgruppen und/oder eine Acylierungsreaktion durchgeführt werden, um die Verbindung der chemischen Formel 1 herzustellen.

**5.** Verfahren nach Anspruch 4,

wobei das Einführen von $^{18}F$ durch ein Verfahren erfolgt, bei dem ein Gemisch von Fluor [$^{18}F$] und TBA in einen Vakuumbehälter eingeführt wird und
Stickstoffgas mit 75 bis 85 °C in den Behälter eingeblasen wird, um das Fluor [$^{18}F$] zu trocknen (Schritt 1); und das im Schritt 1 getrocknet Fluor [$^{18}F$] in ein Reaktionsgefäß eingeführt wird, in dem das Ausgangsmaterial mit der chemischen Formel 2, wie es im Reaktionsschema 1 angegeben ist, und 2,2,6,6-Tetramethylpiperidin-N-oxyl (TEMPO) in einem Lösungsmittel aus Acetonitril/Wasser gelöst sind; gefolgt vom Bestrahlen des Reaktionsgefäßes mit Mikrowellen (Schritt 2).

6. Benzothiazol-Vorläuferverbindungen,
die mit der folgenden chemischen Formel 2 angegeben werden und bei einer Reaktion verwendet werden, um $^{18}F$ direkt einzuführen:

[Chemische Formel 2]

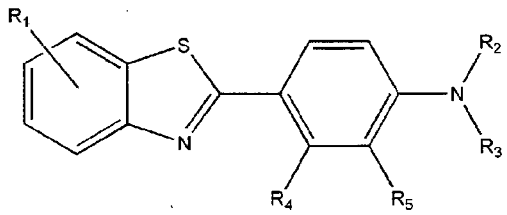

worin $R_6$, $R_{2'}$, $R_{3'}$, $R_{4'}$ und $R_{5'}$ wie im Reaktionsschema 1 von Anspruch 4 angegeben sind.

7. Benzothiazol-Vorläuferverbindungen nach Anspruch 6,
wobei die chemische Verbindung mit der chemischen Formel 2 eine solche ist, die aus der Gruppe ausgewählt ist, bestehend aus
6-Iodphenyl-2-(4'-nitrophenyl)benzothiazoliodoniumtosylat; 6-Iodophenyl-2-(4'-N-methyl(t-butyloxycarbonyl)aminophenyl)benzothiazol-iodoniumtosylat; und 6-Iodphenyl-2-(4'-N,N-dimethylaminophenyl)benzothiazoliodoniumtosylat.

8. Verfahren zum Herstellen von Benzothiazol-Vorläuferverbindungen nach Anspruch 6,
wie es im folgenden Reaktionsschema 3 angegeben ist:

<Reaktionsschema 3>

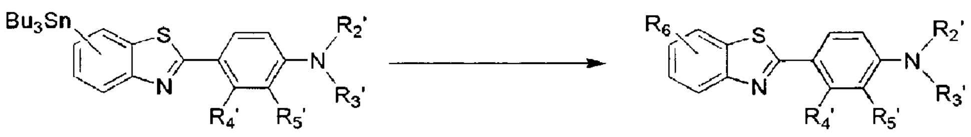

8 2

worin $R_6$, $R_{2'}$, $R_{3'}$, $R_{4'}$ und $R_{5'}$ wie im Reaktionsschema 1 von Anspruch 4 angegeben sind.

9. Verfahren nach Anspruch 8,
wobei der Reaktant zum Einführen des Restes -$R_6$ Hydroxytosyloxyiodbenzol (Koser-Reagenz), 2-Hydroxytosyloxyiodthiophen oder an ein Harz gebundenes 2-Hydroxytosyloxyiodthiophen ist.

10. Verfahren nach Anspruch 8,
wobei die Reaktion durch Auflösen des Reaktanten von Anspruch 9 im Lösungsmittel Acetonitril unter einer Inertgasatmosphäre, Eintropfen der in Methylenchlorid gelösten Verbindung (8) bei 0 °C oder darunter und Rühren der Verbindung für 12 bis 15 Stunden bei Raumtemperatur durchgeführt wird.

11. Bilderzeugungsmittel für die Verwendung bei der Diagnose der Alzheimer-Krankheit,
das die Verbindung nach Anspruch 1 aufweist.

12. Verbindung nach Anspruch 1 für die Verwendung bei der Diagnose des Vorliegens und der Schwere der Alzheimer-Krankheit,
wobei die Verbindung einem Patienten verabreicht wird, bei dem eine Diagnose der Alzheimer-Krankheit erforderlich ist, und das direkte Sichtbarmachen von Beta-Amyloid-Plaques unter Verwendung der Positronenemissionstomographie durch die Bildung einer Bindung der Verbindung mit den Beta-Amyloid-Plaques erfolgt.

## Revendications

1. Composé représenté par la formule chimique 1 ci-dessous :

[Formule chimique 1]

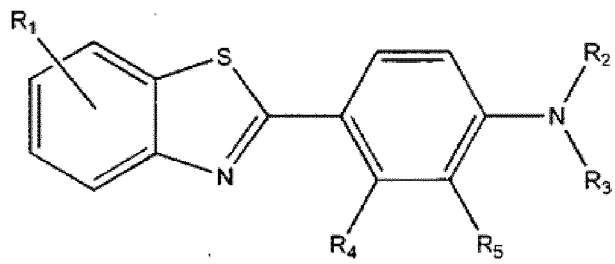

dans laquelle
$R_1$ est $^{18}F$ et $R_1$ est substitué en l'une des positions 4, 5, 6 et 7 du cycle benzothiazole ;
$R_2$ est choisi parmi le groupe comprenant un hydrogène et un alkyle linéaire ou ramifié en $C_1$ à $C_4$, un alkylcarbonyle linéaire ou ramifié en $C_1$ à $C_4$, le 2-(2'-méthoxy-(éthoxy)$_n$)-alkylcarbonyle linéaire ou ramifié en $C_1$ à $C_4$ et le 2-(2'-méthoxy-(éthoxy)$_n$)-alkyle linéaire ou ramifié en $C_1$ à $C_4$, où n est un entier de 1 à 5 ;
$R_3$ est un hydrogène ou un alkyle linéaire ou ramifié en $C_1$ à $C_4$ ; et
$R_4$ et $R_5$ sont chacun indépendamment un hydrogène ou un hydroxy.

2. Composé selon la revendication 1, dans lequel $R_2$ est un hydrogène ou un méthyle, un acétyle, le 2-(2'-méthoxy-(éthoxy)$_n$)acétyle ou le 2-(2'-méthoxy-(éthoxy)$_n$)éthyle, où n est un entier de 1 à 5 ; et $R_3$ est un hydrogène ou un méthyle.

3. Composé selon la revendication 1, dans lequel le composé est choisi parmi le groupe comprenant :

1) le 6-[18F]fluoro-2-(4'-aminophényl)benzothiazole ;
2) le 6-[18F]fluoro-2-(4'-N-méthylaminophényl)benzothiazole ;
et
3) le 6-[18F]fluoro-2-(4'-N,N-diméthylaminophényl)benzothiazole.

4. Méthode de préparation du composé selon la revendication 1, telle qu'indiquée dans la formule réactionnelle 1 suivante, dans laquelle un mélange de [$^{18}$F]fluor et de carbonate de tétrabutylammonium (TBA) est utilisé et mis à réagir avec un composé de formule chimique 2 pour que le cycle benzothiazole soit marqué directement par le $^{18}$F :

<Formule réactionnelle 1>

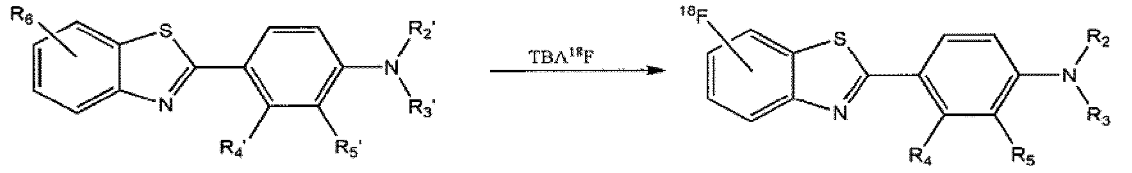


2 1a

dans laquelle,
$R_2$, $R_3$, $R_4$ et $R_5$ sont identiques à ceux définis pour le composé de formule chimique 1 dans la revendication 1 ci-dessus ;
$R_6$ est le iodophényltosylate (

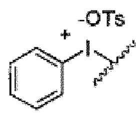

), le 2-iodophénétosylate (

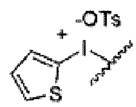

), ou le 2-iodorésinethiophénétosylate (

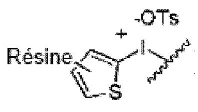

) ;
$R_{2'}$ est l'un des éléments choisi parmi le groupe comprenant les substituants de $R_2$ tels que définis pour le composé de formule chimique 1 dans la revendication 1, et l'oxygène,
$R_{3'}$ est l'un des éléments choisi parmi le groupe comprenant les substituants de $R_3$ tels que définis pour le composé de formule chimique 1 dans la revendication 1, le t-butoxycarbonyle (Boc) et l'oxygène,
et lorsque l'un de $R_{2'}$ et $R_{3'}$ est un hydrogène, l'autre est également un hydrogène, et lorsque $R_{3'}$ est le t-butoxycarbonyle (Boc), $R_3$ est un hydrogène ; et
$R_{4'}$ et $R_{5'}$ sont choisi chacun indépendamment parmi le groupe comprenant un hydrogène et l'éther de méthoxyméthyle (MOM) ; et si nécessaire, avec la mise en oeuvre d'une réaction de réduction, d'alkylation, de déprotection et/ou d'acylation pour produire ledit composé de formule chimique I.

**5.** Méthode selon la revendication 4, dans laquelle l'introduction de $^{18}F$ est effectuée par l'intermédiaire d'un procédé dans lequel un mélange de [$^{18}F$]fluor et de TBA est introduit dans un Vacutainer et de l'azote gazeux est insufflé à une température de 75 °C à 85 °C dans le récipient afin de sécher le [$^{18}F$] fluor (étape 1) ; et le [$^{18}F$] fluor séché dans l'étape 1 est transféré dans une cuve de réaction, dans laquelle un matériau de départ de formule chimique 2, tel que décrit dans la formule réactionnelle 1 et le 2,2,6,6-tétraméthylpipéridine-N-oxyle (TEMPO) sont dissous dans un solvant acétonitrile/eau, opération suivie d'une irradiation de micro-ondes sur la cuve de réaction (étape 2).

**6.** Précurseurs de benzothiazole représentés par la formule chimique 2 suivante, qui sont utilisés dans une réaction destinée à introduire directement du $^{18}F$ :

[Formule chimique 2]

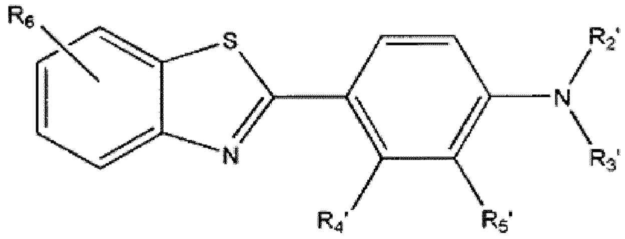

dans laquelle $R_6$, $R_{2'}$, $R_{3'}$, $R_{4'}$ et $R_{5'}$ sont identiques à ceux définis dans la formule réactionnelle 1 de la revendication 4.

**7.** Précurseurs de benzothiazole selon la revendication 6, dans lesquels le composé chimique de formule chimique 2 est l'un des éléments choisi parmi le groupe comprenant :

le tosylate de 6-iodophényl-2-(4'-nitrophényl)benzothiazole-iodonium ;
le tosylate de 6-iodophényl-2-(4'-N-méthyl(t-butyloxycarbonyl)aminophényl)benzothiazole-iodonium ; et
le tosylate de 6-iodophényl-2-(4'-N,N-diméthylaminophényl)-benzothiazole-iodonium.

**8.** Méthode de préparation de précurseurs de benzothiazole selon la revendication 6, telle qu'indiquée dans la formule réactionnelle 3 suivante :

[Formule réactionnelle 3]

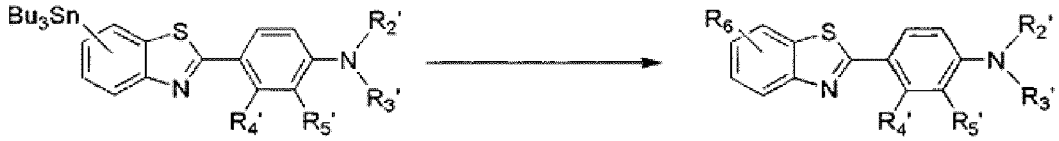

8

2

dans laquelle, $R_6$, $R_{2'}$, $R_{3'}$, $R_{4'}$ et $R_{5'}$ sont identiques à ceux définis dans la formule réactionnelle 1 de la revendication 4.

**9.** Méthode selon la revendication 8, dans laquelle un réactif destiné à l'introduction d'un groupe -$R_6$ est l'hydroxytosyloxyiodobenzène (réactif de Koser), le 2-hydroxytosyloxyiodothiophène ou le 2-hydroxytosyloxyiodothiophène lié à une résine.

**10.** Méthode selon la revendication 8, dans laquelle la réaction est réalisée par dissolution du réactif de la revendication 9 dans du solvant acétonitrile sous une atmosphère de gaz inerte, puis ajout goutte à goutte du composé (8) dissous dans du chlorure de méthylène inférieur ou égal à 0 °C, et agitation du composé à la température ambiante pendant 12 à 15 heures.

**11.** Agent d'imagerie destiné à être utilisé lors du diagnostic de la maladie d'Alzheimer comprenant le composé de la revendication 1.

**12.** Composé selon la revendication 1 destiné à être utilisé pour diagnostiquer la présence et de la sévérité de la maladie d'Alzheimer, dans lequel le composé est administré au sujet requérant un diagnostic de la maladie d'Alzheimer, et une visualisation directe des plaques bêta-amyloïdes est effectuée au moyen d'une tomographie à émission de positons par la formation d'une liaison du composé avec les plaques bêta-amyloïdes.

[Fig. 1]

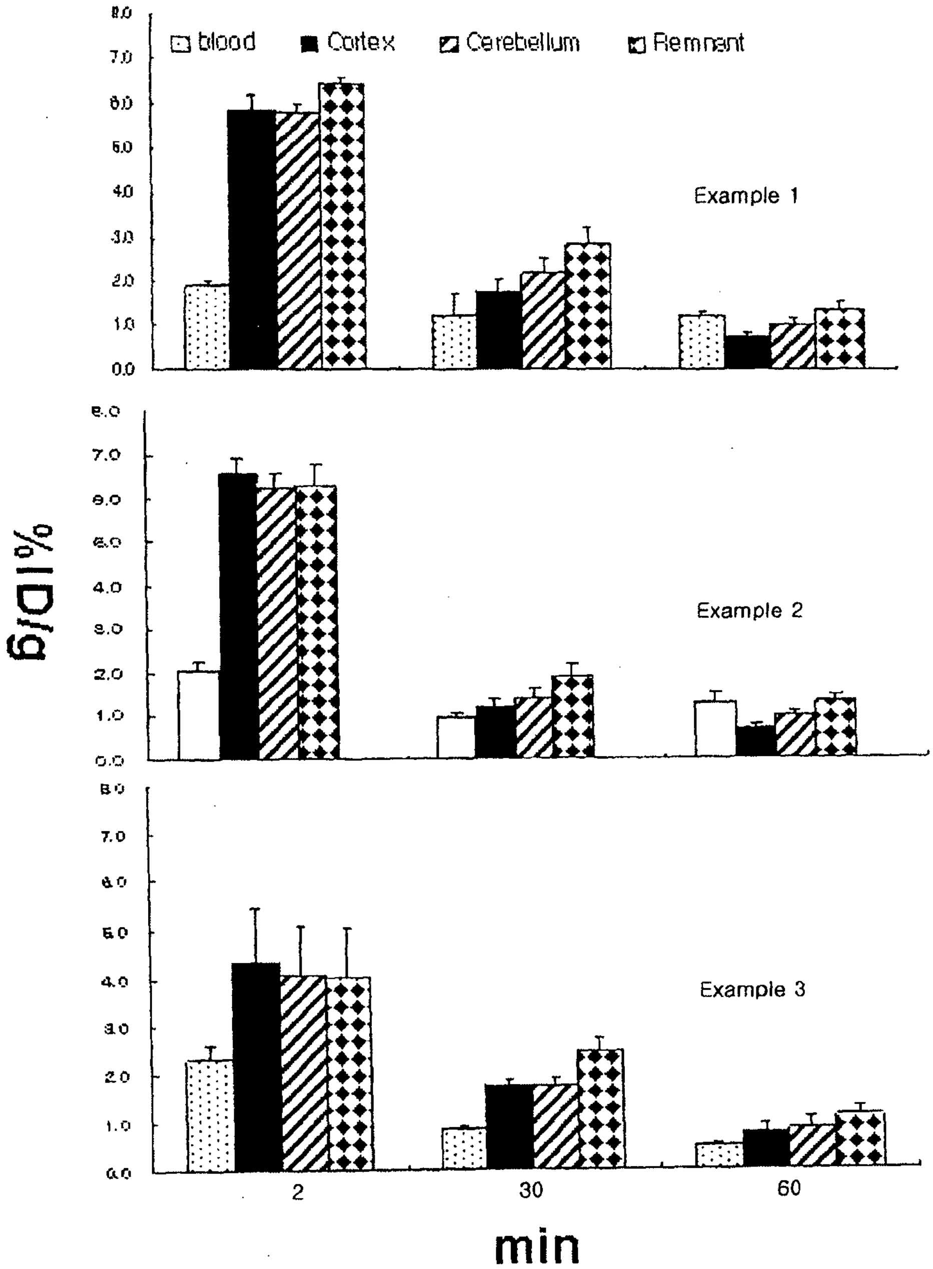
blood
Cortex
Cerebellum
Remnant
Example 1
Example 2
Example 3
%ID/g
min
2
30
60
0.0
1.0
2.0
3.0
4.0
5.0
6.0
7.0
8.0

[Fig. 2]

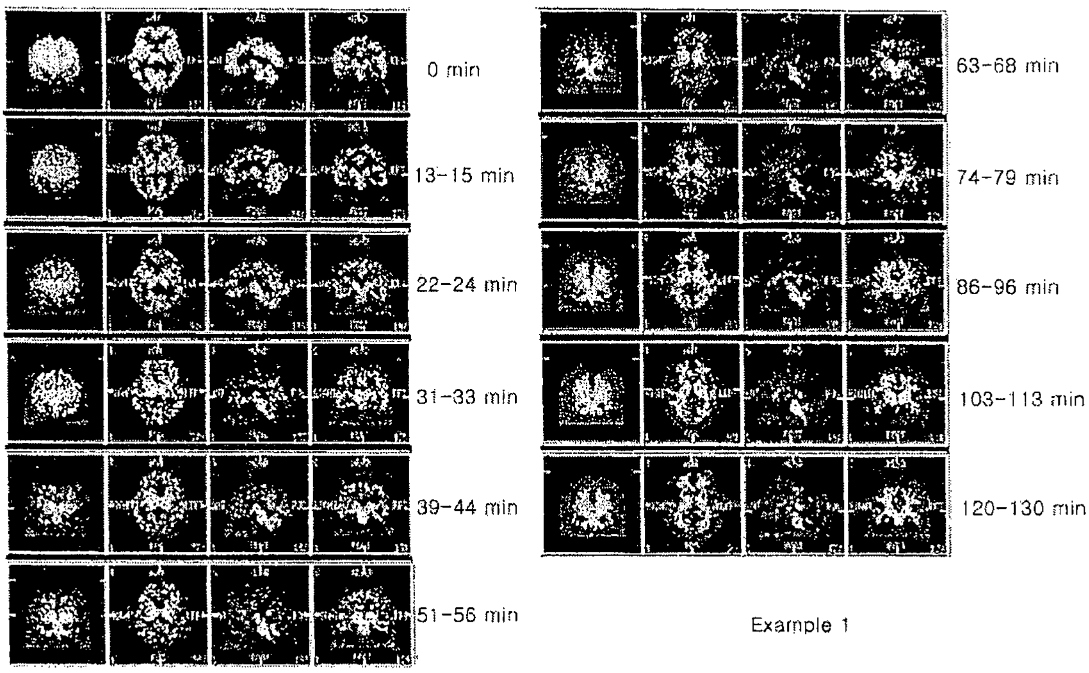
0 min
13-15 min
22-24 min
31-33 min
39-44 min
51-56 min
63-68 min
74-79 min
86-96 min
103-113 min
120-130 min
Example 1

[Fig. 3]

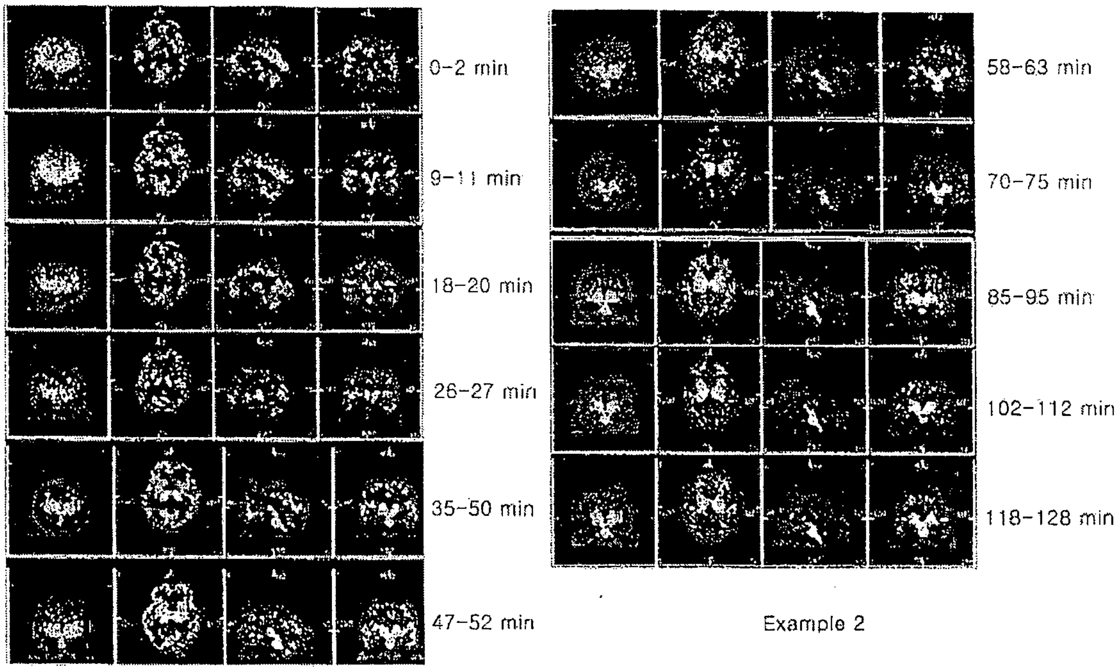
0-2 min
9-11 min
18-20 min
26-27 min
35-50 min
47-52 min
58-63 min
70-75 min
85-95 min
102-112 min
118-128 min
Example 2

[Fig. 4]

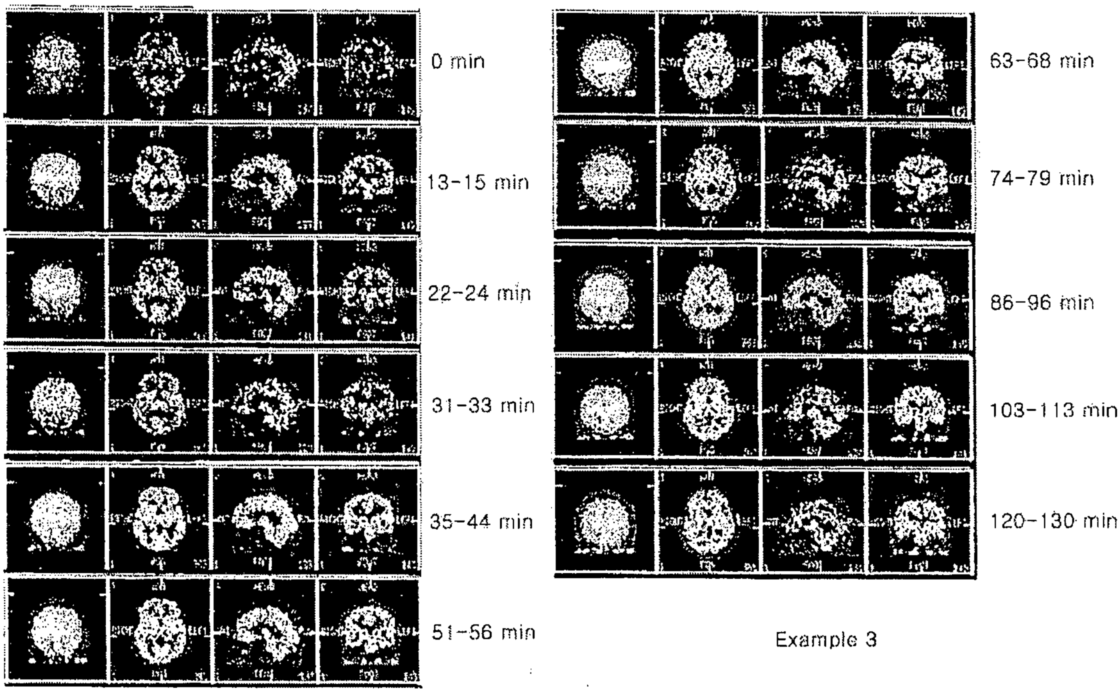
0 min
13-15 min
22-24 min
31-33 min
35-44 min
51-56 min
63-68 min
74-79 min
86-96 min
103-113 min
120-130 min
Example 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MATHIS, C.A. ; WANG. Y. ; HOLT, D. P. ; HUANG, G-F. ; DEBNATH, M.L. ; KLUNK, W.E.** *J. Med. Chem.,* 2003, vol. 46, 2740-2754
- **CAI, L. ; INNIS, R. B. ; PIKE, V. W.** *Curr. Med. Chem.,* 2007, vol. 14 (1), 19-52